(19) Europäisches Patentamt
European Patent Office
Office européen des brevets

(11) EP 0 802 188 B1

(12) **EUROPÄISCHE PATENTSCHRIFT**

(45) Veröffentlichungstag und Bekanntmachung des Hinweises auf die Patenterteilung:
**24.01.2001 Patentblatt 2001/04**

(21) Anmeldenummer: **97105705.4**

(22) Anmeldetag: **07.04.1997**

(51) Int Cl.[7]: **C07D 213/74**, C07D 213/70, C07D 213/75, C07C 233/22, C07D 233/84, C07D 215/36, C07D 235/28, C07D 239/95, A61K 31/44, A61K 31/41, A61K 31/47, A61K 31/165

(54) **Heteroverknüpfte Phenylglycinolamide als antitherosklerotische Arzneimittel**

Phenylglycinolamides linked by a hetero-atom with antiatherosclerotic agents

Phénylglycinolamides reliées par un hétéro-atome ayant une activité antithérosclérotique

(84) Benannte Vertragsstaaten:
**AT BE CH DE DK ES FI FR GB GR IE IT LI LU MC NL PT SE**

(30) Priorität: **18.04.1996 DE 19615262**

(43) Veröffentlichungstag der Anmeldung:
**22.10.1997 Patentblatt 1997/43**

(60) Teilanmeldung: **00109077.8 / 1 028 112**

(73) Patentinhaber: **BAYER AG**
**51368 Leverkusen (DE)**

(72) Erfinder:
- **Goldmann, Siegfried, Dr.**
  **42327 Wuppertal (DE)**
- **Müller, Ulrich, Dr.**
  **42111 Wuppertal (DE)**
- **Connell, Richard, Dr.**
  **Trumbull, CT 06611 (US)**
- **Bischoff, Hilmar, Dr.**
  **42113 Wuppertal (DE)**
- **Denzer, Dirk, Dr.**
  **42115 Wuppertal (DE)**
- **Grützmann, Rudi, Dr.**
  **42657 Solingen (DE)**
- **Beuck, Martin, Dr.**
  **40699 Erkrath (DE)**

(56) Entgegenhaltungen:
**EP-A- 0 344 519**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft heteroverknüpfte Phenylglycinolamide, Verfahren zu ihrer Herstellung und ihre Verwendung als Arzneimittel, insbesondere als antiatherosklerotische Arzneimittel.

Es ist bekannt, daß erhöhte Blutspiegel von Triglyzeriden (Hypertriglyzeridämie) und Cholesterin (Hypercholesterinämie) mit der Genese von atherosklerotischen Gefäßwand-Veränderungen und koronaren Herzkrankheiten assoziiert sind.

EP 344 519 offenbart substituierte 4-(-Chinolin-2-yl-methoxy(phenylessigsäure-Derivate, die als Wirkstoffe in Arzneimitteln eingesetzt werden können. Die Stoffe wirken besonders als Hemmer von enzymatischen Reaktionen im Rahmen des Arachidon-Stoffwechsels, insbesondere der 5-Lipoxygenase.

Ein deutlich erhöhtes Risiko für die Entwicklung koronarer Herzerkrankungen liegt darüber hinaus vor, wenn diese beiden Risikofaktoren kombiniert auftreten, was wiederum mit einer Überproduktion an Apoliprotein B-100 einhergeht. Es besteht daher nach wie vor ein starkes Bedürfnis, wirksame Arzneimittel zur Bekämpfung der Atherosklerose sowie koronarer Herzkrankheiten zur Verfügung zu stellen.

Die vorliegende Erfindung betrifft heteroverknüpfte Phenylglycinolamide der allgemeinen Formel (I)

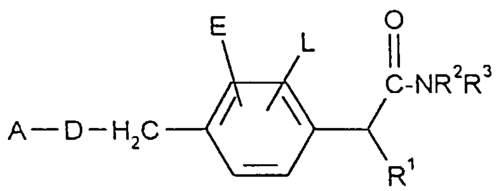

(I)

in welcher

A für Aryl mit 6 bis 10 Kohlenstoffatomen, Benzyl oder für einen gegebenenfalls benzokondensierten 5- bis 7-gliedrigen gesättigten partiell ungesättigten oder ungesättigten Heterocyclus mit bis zu 3 Heteroatomen aus der Reihe S, N und/oder O steht, wobei die Ringsysteme gegebenenfalls - auch über die N-Funktion - bis zu 3-fach gleich oder verschieden durch Halogen, Trifluormethyl, Carboxy, Hydroxyl, Nitro, Cycloalkyl mit 3 bis 7 Kohlenstoffatomen, Benzyl, Phenyl, Benzyloxy oder durch geradkettiges oder verzweigtes Alkyl, Alkoxy oder Alkoxycarbonyl mit jeweils bis zu 6 Kohlenstoffatomen substituiert sind, oder für einen Rest der Formel $R^5R^4N$- oder

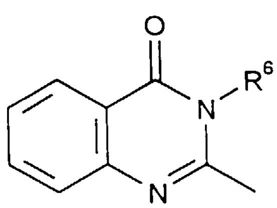

steht,
worin

$R^4$ und $R^5$ gleich oder verschieden sind und Wasserstoff, Phenyl oder geradkettiges oder verzweigtes Alkyl mit bis zu 6 Kohlenstoffatomen bedeuten,

$R^6$ Wasserstoff, Phenyl oder geradkettiges oder verzweigtes Alkyl mit bis zu 4 Kohlenstoffatomen bedeutet,

D für ein Sauerstoffatom oder für einen Rest der Formel -CO-, $-(CO)_a-NR^7$, $-(CH_2)_bS-$, $-(CH_2)_c-NR^8$ oder -CH=CH- steht,
worin

a, b und c gleich oder verschieden sind und eine Zahl 0 oder 1 bedeuten,

$R^7$ und $R^8$ gleich oder verschieden sind und Wasserstoff, geradkettiges oder verzweigtes Alkyl oder Acyl mit jeweils bis zu 6 Kohlenstoffatomen, Phenyl oder Benzyl bedeuten, wobei die Ringsysteme gegebenenfalls bis zu 2-fach gleich oder verschieden durch Nitro, Halogen, Trifluormethyl oder durch geradkettiges oder verzweigtes Alkyl oder Alkoxy mit jeweils bis zu 3 Kohlenstoffatomen substituiert sind,

E und L gleich oder verschieden sind und
für Wasserstoff, Cycloalkyl mit 3 bis 8 Kohlenstoffatomen, Azido, Hydroxy, Halogen, geradkettiges oder verzweigtes Alkyl, Alkoxy oder Alkenyl mit jeweils bis zu 6 Kohlenstoffatomen stehen,

$R^1$ für Cycloalkyl mit 3 bis 8 Kohlenstoffatomen steht, oder für geradketiges oder verzweigtes Alkyl mit bis zu 10 Kohlenstoffatomen steht,

$R^2$ für Wasserstoff oder für geradkettiges oder verzweigtes Alkyl mit bis zu 4 Kohlenstoffatomen steht,

$R^3$ für einen Rest der Formel

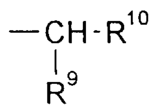

steht,
worin

$R^9$ Wasserstoff oder einen Rest der Formel $CH_2$-OH bedeutet,

$R^{10}$ Phenyl bedeutet, das gegebenenfalls bis zu 3-fach gleich oder verschieden durch Hydroxy, Halogen oder geradkettiges oder verzweigtes Alkyl mit bis zu 5 Kohlenstoffatomen substituiert ist,

und deren Salze.

Die erfindungsgemäßen heteroverknüpften Phenylglycinolamide können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di-bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren als auch Diastereomeren oder deren jeweiligen Mischungen. Diese Mischungen der Enantiomeren und Diastereomeren lassen sich in bekannter Weise in die stereoisomer einheitlichen Bestandteile trennen.

Heterocyclus, gegebenenfalls benzokondensiert, steht im Rahmen der Erfindung im allgemeinen für einen gesättigten, partiell ungesättigten oder ungesättigten 5-bis 7-gliedrigen, vorzugsweise 5- bis 6-gliedrigen Heterocyclus der bis zu 3 Heteroatome aus der Reihe S, N und/oder O enthalten kann und der im Fall eines Stickstoffatoms auch über dieses gebunden sein kann. Beispielsweise seien genannt: Indolyl, Chinolyl, Benzo[b]thienyl, Benzo[b]furyl, Pyridyl, Thienyl, Furyl, Pyrrolyl, Thiazolyl, Oxazolyl, Imidazolyl, Morpholinyl oder Piperidyl. Bevorzugt sind Chinolyl, Furyl, Pyridyl und Thienyl.

Bevorzugt sind die erfindungsgemäßen Verbindungen der allgemeinen Formel (I), in welcher

A für Naphthyl, Phenyl, Benzyl, Pyridyl, Imidazolyl, Benzimidazolyl oder Chinolyl steht, die gegebenenfalls -

auch über die N-Funktion- bis zu 3-fach gleich oder verschieden durch Fluor, Chlor, Brom, Trifluormethyl, Carboxy, Hydroxyl, Nitro, Cyclopropyl, Cylcopentyl, Cyclohexyl, Benzyl, Phenyl, Benzyloxy oder durch geradkettiges oder verzweigtes Alkyl, Alkoxy oder Alkoxycarbonyl mit jeweils bis zu 4 Kohlenstoffatomen substituiert sind, oder
für einen Rest der Formel $R^5R^4N$- oder

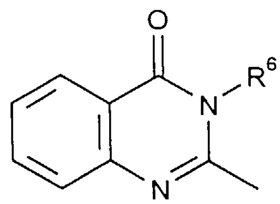

steht, worin

$R^4$ und $R^5$ gleich oder verschieden sind und Wasserstoff, Phenyl oder geradkettiges oder verzweigtes Alkyl mit bis zu 4 Kohlenstoffatomen bedeuten,

$R^6$ Wasserstoff, Phenyl oder geradkettiges oder verzweigtes Alkyl mit bis zu 3 Kohlenstoffatomen bedeutet,

D für ein Sauerstoffatom oder für einen Rest der Formel -CO-, $-(CO)_a-NR^7$, $-(CH_2)_bS-$, $-(CH_2)_c-NR^8$ oder -CH=CH- steht,
worin

a, b und c gleich oder verschieden sind und eine Zahl 0 oder 1 bedeuten,

$R^7$ und $R^8$ gleich oder verschieden sind und Wasserstoff, geradkettiges oder verzweigtes Alkyl oder Acyl mit jeweils bis zu 5 Kohlenstoffatomen, Phenyl oder Benzyl bedeuten, wobei die Ringsysteme gegebenenfalls bis zu 2-fach gleich oder verschieden durch Nitro, Fluor, Chlor, Brom, Trifluormethyl oder durch geradkettiges oder verzweigtes Alkyl oder Alkoxy mit jeweils bis zu 3 Kohlenstoffatomen substituiert sind,

E und L gleich oder verschieden sind und für Wasserstoff, Cyclopropyl, Cyclopentyl, Cyclohexyl, Azido, Hydroxy, Fluor, Chlor, Brom, geradkettiges oder verzweigtes Alkyl, Alkoxy oder Alkenyl mit jeweils bis zu 3 Kohlenstoffatomen stehen,

$R^1$ für Cyclopropyl, Cylcobutyl, Cyclopentyl, Cyclohexyl oder Cycloheptyl steht, oder für geradkettiges oder verzweigtes Alkyl mit bis zu 8 Kohlenstoffatomen steht,

$R^2$ für Wasserstoff oder für geradkettiges oder verzweigtes Alkyl mit bis zu 3 Kohlenstoffatomen steht,

$R^3$ für einen Rest der Formel

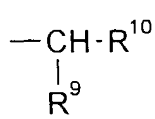

steht, worin

$R^9$ Wasserstoff oder einen Rest der Formel $CH_2$-OH bedeutet,

$R^{10}$ Phenyl bedeutet, das gegebenenfalls bis zu 2-fach gleich oder verschieden durch Hydroxy, Fluor, Chlor, Brom oder geradkettiges oder verzweigtes Alkyl mit bis zu 3 Kohlenstoffatomen substituiert

ist,

und deren Salze.

Besonders bevorzugt sind die erfindungsgemäßen Verbindungen der allgemeinen Formel (I), in welcher

A für Naphthyl, Phenyl, Benzyl, Pyridyl, Imidazolyl, Benzimidazolyl oder Chinolyl steht, die gegebenenfalls - auch über die N-Funktion- bis zu 2-fach gleich oder verschieden durch Fluor, Chlor, Trifluormethyl, Carboxy, Hydroxyl, Nitro, Cyclohexyl, Benzyl, Phenyl, Benzyloxy oder durch geradkettiges oder verzweigtes Alkyl, Alkoxy oder Alkoxycarbonyl mit jeweils bis zu 3 Kohlenstoffatomen substituiert sind, oder für einen Rest der Formel $R^5R^4N$- oder

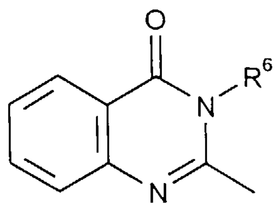

steht, worin

$R^4$ und $R^5$ gleich oder verschieden sind und Wasserstoff, Phenyl oder Methyl bedeuten,

$R^6$ Wasserstoff, Phenyl oder Methyl bedeutet,

D für ein Sauerstoffatom oder für einen Rest der Formel -CO-, $-(CO)_a-NR^7$, $-(CH_2)_bS-$, $-(CH_2)_c-NR^8$ oder -CH=CH- steht, worin

a, b und c gleich oder verschieden sind und eine Zahl 0 oder 1 bedeuten,

$R^7$ und $R^8$ gleich oder verschieden sind und Wasserstoff, Methyl, Ethyl, Phenyl, Acetyl oder Benzyl bedeuten, wobei die Ringsysteme gegebenenfalls bis zu 2-fach gleich oder verschieden durch Nitro, Fluor, Chlor, Brom, Trifluormethyl oder durch geradkettiges oder verzweigtes Alkyl oder Alkoxy mit jeweils bis zu 3 Kohlenstoffatomen substituiert sind,

E und L gleich oder verschieden sind und für Wasserstoff, Fluor, Chlor oder Brom stehen,

$R^1$ für Cyclopropyl, Cylcobutyl, Cyclopentyl oder Cyclohexyl steht, oder für geradkettiges oder verzweigtes Alkyl mit bis zu 8 Kohlenstoffatomen steht,

$R^2$ für Wasserstoff oder für geradkettiges oder verzweigtes Alkyl mit bis zu 3 Kohlenstoffatomen steht,

$R^3$ für einen Rest der Formel

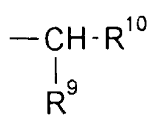

steht, worin

$R^9$ Wasserstoff oder einen Rest der Formel $CH_2$-OH bedeutet,

$R^{10}$ Phenyl bedeutet, das gegebenenfalls bis zu 2-fach gleich oder verschieden durch Hydroxy, Fluor,

Chlor, Methyl oder Ethyl substituiert ist,

und deren Salze.

Außerdem wurde ein Verfahren zur Herstellung der erfindungsgemäßen Verbindungen der allgemeinen Formel (I) gefunden, dadurch gekennzeichnet, daß man

Carbonsäuren der allgemeinen Formel (II),

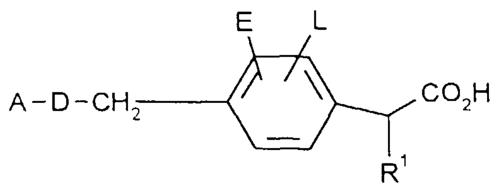

(II)

in welcher

A, D, E, L und $R^1$ die oben angegebene Bedeutung haben,

gegebenenfalls unter vorgeschalteter Aktivierung der Carbonsäurefunktion, mit Phenylglycinolen der allgemeinen Formel (III)

$$HR^2N\text{-}R^3 \qquad \text{(III)}$$

in welcher

$R^2$ und $R^3$ die oben angegebene Bedeutung haben,

gegebenenfalls unter Schutzgasatmosphäre, gegebenenfalls in inerten Lösemitteln, in Anwesenheit einer Base und/ oder Hilfsmittels umsetzt.

Das erfindungsgemäße Verfahren kann durch folgendes Formelschema beispielhaft erläutert werden:

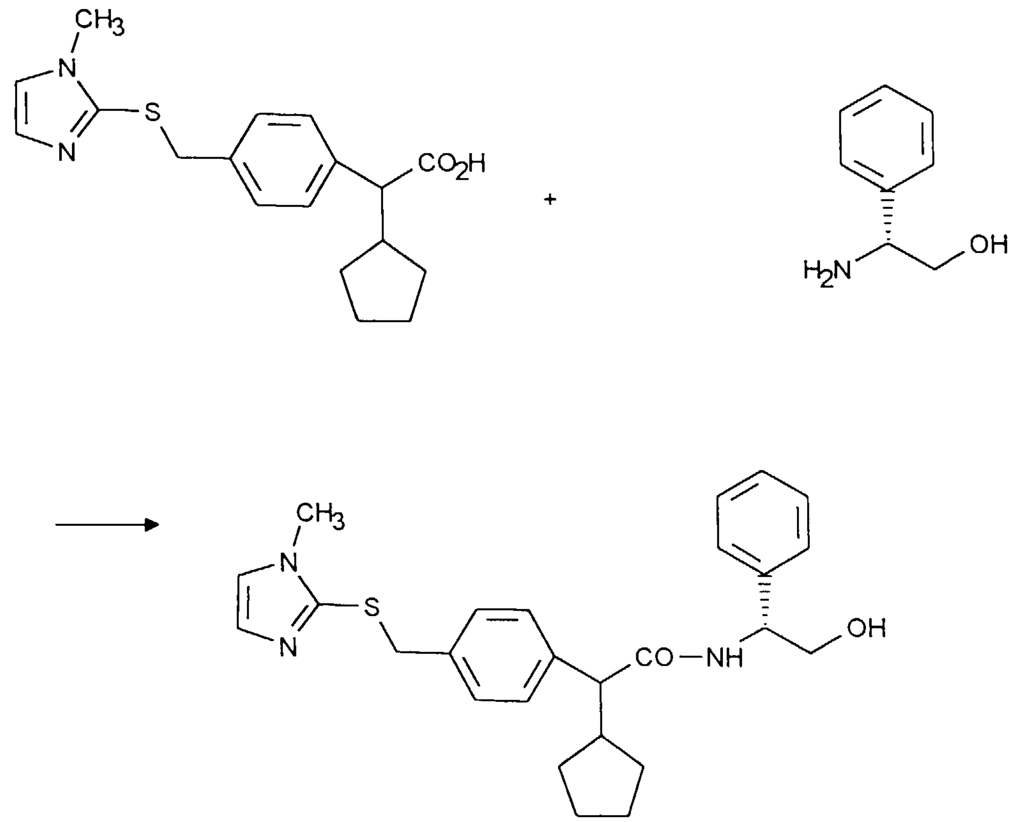

Als Lösemittel eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Dichlormethan, Tetrahydrofuran, Aceton und Dimethylformamid.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliumethanolat, oder Natrium- oder Kaliummethanolat, oder organische Amine wie Triethylamin, Picolin oder N-Methylpiperidin, oder Amide wie Natriumamid oder Lithiumdiisopropylamid, oder metallorganische Verbindungen wie Butyllithium oder Phenyllithium. Bevorzugt sind Natrium- und Kaliumcarbonat und Triethylamin.

Die Base wird in einer Menge von 0,6 mol bis 5 mol, bevorzugt von 0,7 mol bis 2 mol, bezogen auf 1 mol der Verbindung der allgemeinen Formel (II) eingesetzt.

Die Reaktion wird im allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Zur Aktivierung der Carbonsäurefunktion eignen sich im allgemeinen Basen und/oder Dehydratisierungsreagenzien wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphorsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Verbindungen der allgemeinen Formel (II) sind teilweise bekannt oder neu und können beispielsweise

hergestellt werden, indem man

Verbindungen der allgemeinen Formel (IV)

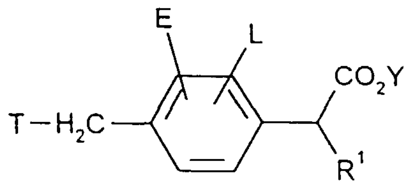

(IV)

in welcher

| | |
|---|---|
| E, L und $R^1$ | die oben angegebene Bedeutung haben, |
| T | für eine typische Abgangsgruppe wie z.B. Chlor, Brom, Jod, Tosylat oder Mesylat, vorzugsweise für Brom steht, und |
| Y | für Wasserstoff, für $(C_1-C_4)$-Alkyl, oder eine andere basenstabile Schutzgruppe steht, |

mit Verbindungen der allgemeinen Formel (V)

A-H (V)

in welcher
A die oben angegebene Bedeutung hat
in inerten Lösemitteln, gegebenenfalls in Anwesenheit einer Base umsetzt,
und anschließend die Ester oder die Schutzgruppe Y nach üblichen Methoden abspaltet.

Die Verbindungen der allgemeinen Formeln (IV) und (V) sind an sich bekannt oder nach üblichen Methoden herstellbar.

Die Verbindungen der allgemeinen Formel (III) sind ebenfalls bekannt oder nach üblichen Methoden herstellbar.

Die erfindungsgemäßen Verbindungen der allgemeinen Formel (I) haben ein nicht vorhersehbares pharmakologisches Wirkspektrum.

Sie können als Wirkstoffe in Arzneimitteln zur Reduzierung von Veränderungen an Gefäßwänden Verwendung finden und zur Behandlung von Koronaren Herzerkrankungen, Herzinsuffizienz, Störungen der Hirnleistung, ischämischen Gehirnerkrankungen, Apoplexie, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die erfindungsgemäßen Verbindungen zeichnen sich durch eine Senkung der ApoB-100-assoziierten Lipoproteinen (VLDL und seiner Abbauprodukte, wie z.B. LDL), des ApoB-100, der Triglyceride und des Cholesterins aus. Damit besitzen sie wertvolle, im Vergleich zum Stand der Technik überlegene pharmakologische Eigenschaften.

Überraschenderweise besteht die Wirkung der erfindungsgemäßen Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muß mit einer Senkung der Plasmaspiegel von ApoB-100, LDL, Triglyceriden und von Cholesterin einhergehen; es werden also gleichzeitig mehrere der obengenannten Risikofaktoren gesenkt, die an Gefäßwandveränderungen beteiligt sind.

Die erfindungsgemäßen Verbindungen können daher zur Präventation und Behandlung von Atherosklerose, der Fettsucht, Pankreatitis und der Obstipation eingesetzt werden.

## 1. Hemmung der Freisetzung ApoB-100-assoziierter Lipoproteine

Der Test zum Nachweis der Hemmung der Freisetzung ApoB-100-assoziierter Liproproteine aus Leberzellen erfolgte in vitro mit kultivierten Leberzellen, bevorzugt mit Zellen der humanen Linie HepG2. Diese Zellen werden unter

Standardbedingungen in Medium für die Kultur eukariontischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL- bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorbtion an die Plastikoberfläche von Mikrotitierplatten mit 96 Vertiefungen, bevorzugt an MaxiSorp-Platten. Wenn im Überstand von Hep-G2-Zellen ApoB-100-assoziierte Partikel vorhanden sind, dann können diese an die insolubilisierten Kan-anti-LDL-Ak binden, und es entsteht ein Immunkomplex, der an die Plastikoberfläche gebunden ist. Nicht gebundene Proteine werden durch Waschen entfernt. Der sich an der Plastikoberfläche befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase konjugiert. Peroxidase setzt das farblose Substrat TMB in Gegenwart von $H_2O_2$ in ein gefärbtes Produkt um. Nach Ansäuerung des Reaktionsgemisches mit $H_2SO_4$ wird die spezifische Lichtadsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die erfindungsgemäßen Verbindungen die Freisetzung der ApoB-100-assoziierten Partikel. Der $IC_{50}$-Wert gibt an, bei welcher Substanzkonzentration die Lichtadsorption im Vergleich zur Kontrolle (Lösemittelkontrolle ohne Substanz) um 50% inhibiert ist.

**2. Bestimmung der VLDL-Sekretion in vivo am Hamster**

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i. p.) narkotisiert. Wenn die Tiere reflexfrei geworden sind, wird die V. jugularis freipräpariert und kanüliert. Anschließend werden 0,25 ml/kg einer 20%igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g zentrifugiert. Im so erhaltenen Serum wird die Triglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-Lochplatten versetzt und 10 Minuten bei Raumtemperatur inkubiert. Anschließend wird die optische Dichte bei einer Wellenlänge von 492 nm in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in diesem Modell entweder unmittelbar vor Applikation des Detergens intravenös verabreicht oder vor Einleitung der Narkose oral oder subkutan.

**3. Hemmung der intestinalen Triglyceridabsorption in vivo (Ratten)**

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird ihnen das Futter entzogen. Trinkwasser steht den Tieren ad libitum zur Verfügung. Die Tiere der Kontrollgruppen erhalten eine wäßrige Traganth-Suspension bzw. eine Traganth-Suspension die Olivenöl enthält. Die Traganth-Olivenöl-Suspension wird mit dem Ultra-Turrax hergestellt. Die zu untersuchenden Substanzen werden in einer entsprechenden Traganth-Olivenöl-Suspension ebenfalls mit dem Ultra-Turrax, direkt vor der Substanzapplikation suspendiert.

Jeder Ratte wird vor der Schlundsondenapplikation zur Bestimmung des basalen Serumtriglyceridgehaltes Blut durch Punktion des retroorbitalen Venenplexus' entnommen. Anschließend werden die Traganth-Suspension, die Traganth-Olivenöl-Suspensionen ohne Substanz (Kontrolltiere), bzw. die Substanzen, suspendiert in einer entsprechenden Traganth-Olivenöl-Suspension, den nüchternen Tieren mit einer Schlundsonde verabreicht. Die weiteren Blutentnahmen zur Bestimmung des postprandialen Serumtriglyceridanstiegs erfolgen in der Regel 1, 2 und 3 Stunden nach der Schlundsondenapplikation.

Die Blutproben werden zentrifugiert und nach Gewinnung des Serums die Triglyceride photometrisch mit einem EPOS-Analyzer 5060 (Eppendorf Gerätebau, Netheler & Hinz GmbH, Hamburg) bestimmt. Die Bestimmung

der Trigylceride erfolgt vollenzymatisch mit einem handelsüblichen UV-Test.

Der postprandiale Serumtriglyceridanstieg wird durch Subtraktion des Triglyceridvorwertes jeden Tieres von seinen korrespondierenden postprandialen Triglyceridkonzentrationen (1, und 3 Stunden nach Applikation) ermittelt.

Die Differenzen (in mmol/1) zu jedem Zeitpunkt (1, 2 und 3 Stunden) werden in den Gruppen gemittelt, und die Mittelwerte des Serumtriglyceridanstiegs (ΔTG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in Δ% von der ölbelasteten Kontrolle angegeben.

$$\Delta\% \text{ Triglyceridanstieg} = \frac{\Delta TG_{Substanz} - \Delta TG_{Traganthkontrolle}}{\Delta TG_{Ölbelastung} - \Delta TG_{Traganthkontrolle}} \times 100$$

Effekt von 10 mg Prüfsubstanz / kg KG p.o. auf den Triglyceridanstieg (Δ%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant (p <0,05) um mindestens 30 % vermindern, werden als pharmakologisch wirksam angesehen.

**4. Hemmung der VLDL-Sekretion in vivo (Ratte)**

Die Wirkung der Testsubstanzen auf die VLDL-Sekretion wird ebenfalls an der Ratte untersucht. Dazu wird Ratten 500 mg/kg Körpergewicht (2,5 mg/kg) Triton WR-1339, gelöst in physiologischer Kochsalzlösung, intravenös in die Schwanzvene appliziert. Triton WR-1339 inhibiert die Lipoproteinlipase und führt somit durch Hemmung des VLDL-Katabolismus zu einem Anstieg des Triglycerid- und Cholsterinspiegels. Diese Anstiege können als Maß für die VLDL-Sekretionsrate herangezogen werden.

Den Tieren wird vor sowie eine und zwei Stunden nach Applikation des Detergens durch Punktion des retroorbitalen Venenplexus Blut entnommen. Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Meßbereich der Methoden überschreiten, werden mit physiologischer Kochsalzlösung verdünnt. Die Ermittlung der jeweiligen Serumkonzentrationen erfolgt anhand parallel gemessener Standardreihen. Testsubstanzen werden unmittelbar nach der Tritoninjektion oral, intravenös oder subcutan appliziert.

Die Erfindung betrifft außerdem die Kombination von heteroverknüpften Phenylglycinolamiden der allgemeinen Formel (I) mit einem Glucosidase- und/oder Amylasehemmer zur Behandlung von familiärer Hyperlipidaeamien, der Fettsucht (Adipositas) und des Diabetes mellitus. Glucosidase- und/oder Amylasehemmer im Rahmen der Erfindung sind beispielsweise Acarbose, Adiposine, Voglibose, Miglitol, Emiglitate, MDL-25637, Camiglibose (MDL-73945), Tendamistate, AI-3688, Trestatin, Pradimicin-Q und Salbostatin.

Bevorzugt ist die Kombination von Acarbose, Miglitol, Emiglitate oder Voglibose mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter

flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

**Abkürzungen im experimentellen Teil**

Bn = $CH_2$-$C_6H_5$

Gly =

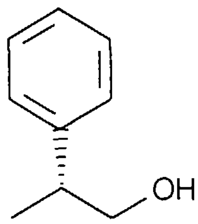

Me = Methyl

Et = Ethyl

cHex = Cyclohexyl

Ph = Phenyl

**Lösemittel**

C/EE = Cyclohexan / Essigester

P/EE = Petrolether / Essigester

**Ausgangsverbindungen**

**Beispiel I**

N-(2-Chlorbenzoyl)-2-cyclopentyl-4-(phenylaminomethyl)-phenyl-essigsäure-tert.butylester

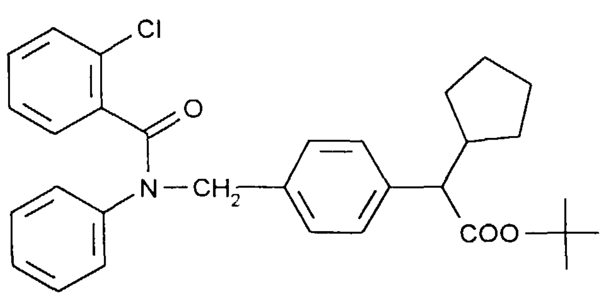

2,3 g (10 mmol) 2-Chlorbenzoesäureanilid werden in 10 ml DMF gelöst, mit 330 mg (11 mmol) NaH (80%ig) deprotoniert

(50°C) und anschließend mit 3,5 g (10 mmol) 4-Brommethyl-2-cyclopentyl-essigsäure-tert.butylester (DE 42 00 954 A1) versetzt und über Nacht bei RT gerührt. Es wird eingeengt, in $CH_2Cl_2$ gelöst, mit $H_2O$ gewaschen, eingeengt und der Rückstand an Kieselgel chromatographiert (Cylcohexan / EE = 8:2).
Man erhält 3,8 g (75%) als farbloses Harz.

**Beispiel II**

N-(2-Chlorbenzoyl)-2-cyclopentyl-4-(phenylaminomethyl)-phenyl-essigsäure

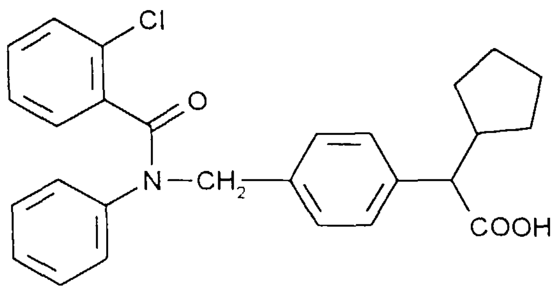

3,5 g (7 mmol) der Verbindung aus Beispiel I werden in 14 ml Dioxan gelöst, mit 2 ml konz. HCl versetzt und 5 h refluxiert. Es wird eingeengt, in $CH_2Cl_2$ aufgenommen und mit Wasser gewaschen. Man erhält 3 g (96%) der Titelverbindung als Öl.
In Analogie zur Vorschrift des Beispiels II werden die in der Tabellen I - III aufgeführten Beispiele hergestellt.

**Tabelle I:**

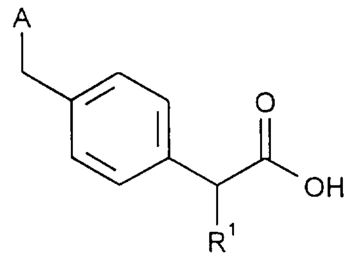

| Bsp.-Nr. | A | $R^1$ | Fp. (°C) | $R_f$ |
|---|---|---|---|---|
| III | 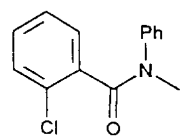 | (R&S) cPent | | 0,28<br>C/EE 1:1 |
| IV | 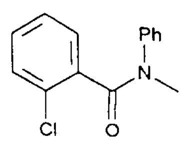 | (R&S) cHept | | 0,3<br>C/EE 1:1 |
| V | 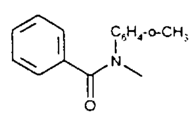 | (R&S) cPent | | 0,32<br>C/EE 1:1 |
| VI | 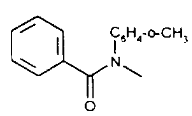 | (R&S) cHept | | 0,46<br>C/EE 1:1 |
| VII | 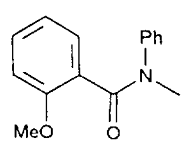 | (R&S) cPent | | 0,23<br>C/EE 1:1 |

| Bsp.-Nr. | A | $R^1$ | Fp. (°C) | $R_f$ |
|---|---|---|---|---|
| VIII | 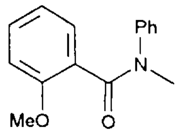 | (R&S) cHept | | 0,19 C/EE 1:1 |
| IX | 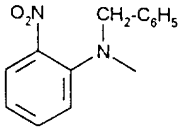 | (R&S) cPent | | 0,22 C/EE 1:1 |
| X | 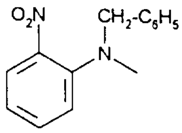 | (R&S) cHept | | 0,28 C/EE 1:1 |
| XI | 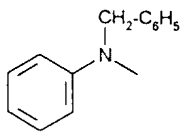 | (R&S) cPent | | 0,25 C/EE 1:1 |
| XII | 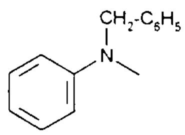 | (R&S) cHept | | 0,33 C/EE 1:1 |
| XIII | 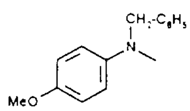 | (R&S) cPent | | 0,29 C/EE 1:1 |
| XIV | 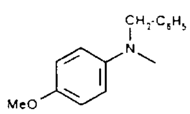 | (R&S) cHept | | 0,35 C/EE 1:1 |
| XV | 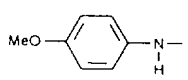 | (R&S) cPent | 151 | |
| XVI | 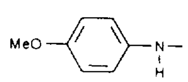 | (R&S) cHept | 155 | |

| Bsp.-Nr. | A | $R^1$ | Fp. (°C) | $R_f$ |
|---|---|---|---|---|
| XVII | 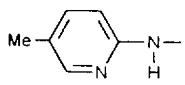 | (R&S) cPent | | 0,05<br>C/EE 1:1 |
| XVIII | 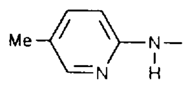 | (R&S) cHept | | 0,05<br>C/EE 1:1 |
| XIX | 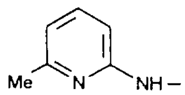 | (R&S) cPent | | 0,11<br>($CH_2Cl_2/CH_3OH$ 95:5) |
| XX | 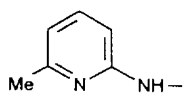 | (R&S) cHept | 211 | |
| XXI | 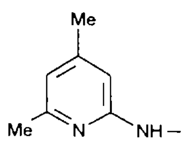 | (R&S) cPent | 211 | |
| XXII | 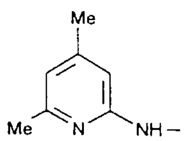 | (R&S) cHept | 190 | |
| XXIII | 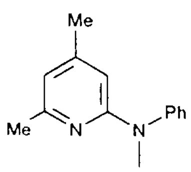 | (R&S) cPent | | 0,58<br>C/EE 1:1 |
| XXIV | 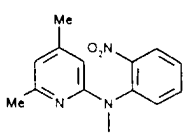 | (R&S) cPent | | 0,11<br>C/EE 7:3 |

| Bsp.-Nr. | A | $R^1$ | Fp. (°C) | $R_f$ |
|---|---|---|---|---|
| XXV | 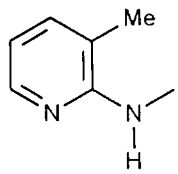 | (R&S) cPent | | 0,13 C/EE 1:1 |
| XXVI | 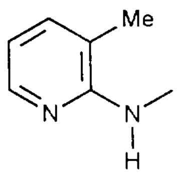 | (R&S) cHept | 244 | |
| XXVII | 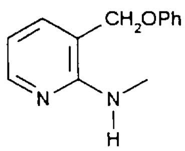 | (R&S) cPent | 159 | |
| XXVIII | 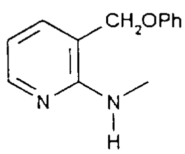 | (R&S) cHept | | 0,06 C/EE 7:3 |
| XXIX | 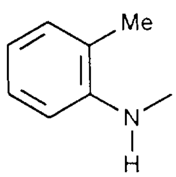 | (R&S) cPent | | |
| XXX | 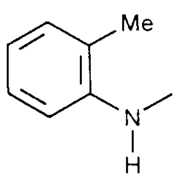 | (R&S) cHept | | 0,15 C/EE 7:3 |

| Bsp.-Nr. | A | $R^1$ | Fp. (°C) | $R_f$ |
|---|---|---|---|---|
| XXXI | 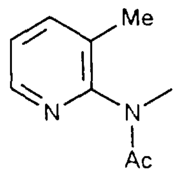 | (R&S) cHept | | |
| XXXII | 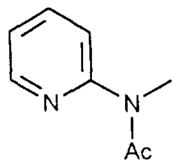 | (R&S) cPent | | |
| XXXIII | 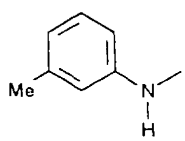 | (R&S) cPent | | 0,42 C/EE 1:1 |
| XXXIV | 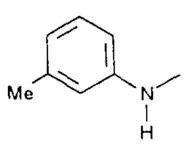 | (R&S) cHept | | 0,15 C/EE 7:3 |
| XXXV | 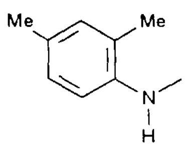 | (R&S) cPent | | 0,13 C/EE 7:3 |
| XXXVI | 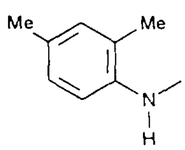 | (R&S) cHept | | 0,18 C/EE 7:3 |

**Tabelle II:**

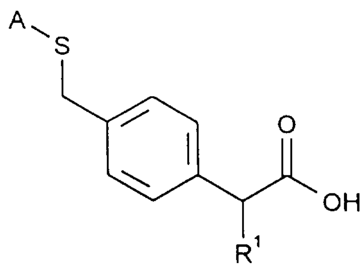

| Bsp.-Nr. | A | $R^1$ | Fp. (°C) | $R_f$ |
|---|---|---|---|---|
| XXXVII | 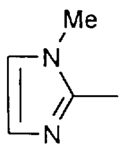 | (R&S) cPent | 158 | |
| XXXVIII | 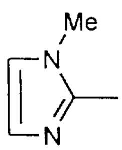 | (R&S) cHept | | 0,1<br>C/EE 3:7 |
| XXXIX | 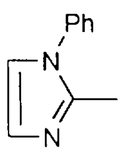 | (R&S) cPent | 184 | |
| XL | 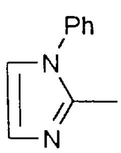 | (R&S) cHept | 139 | |
| XLI | 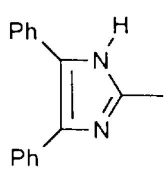 | (R&S) cPent | | 0,27<br>C/EE 1:1 |

| Bsp.-Nr. | A | $R^1$ | Fp. (°C) | $R_f$ |
|---|---|---|---|---|
| XLII | 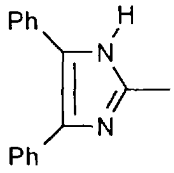 | (R&S) cHept | 172 | |
| XLIII | 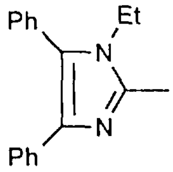 | (R&S) cPent | 205 | |
| XLIV | 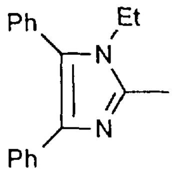 | (R&S) cHept | 190 | |
| XLV | 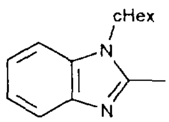 | (R&S) cPent | 197 | |
| XLVI | 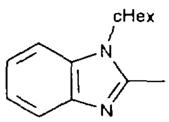 | (R&S) cHept | 213 | |
| XLVII | 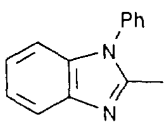 | (R&S) cPent | 204 | |
| XLVIII | 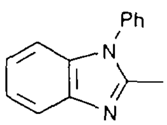 | (R&S) cHept | 205 | |

| Bsp.-Nr. | A | $R^1$ | Fp. (°C) | $R_f$ |
|---|---|---|---|---|
| XLIX | 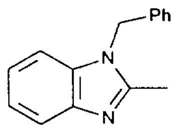 | (R&S) cPent | 173 | |
| L | 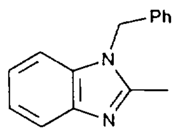 | (R&S) cHept | 146 | |
| LI | 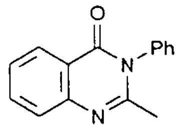 | (R&S) cPent | | 0,3<br>C/EE 7:3 |
| LII | 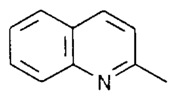 | (R&S) cPent | 119 | |
| LIII | 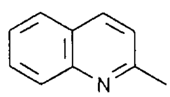 | (R&S) cHept | | 0,21<br>C/EE 7:3 |
| LIV | 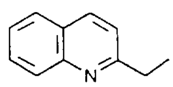 | (R&S) cPent | 136 | |
| LV | 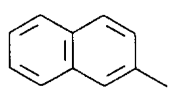 | (R&S) cPent | 129 | |
| LVI | 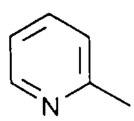 | (R&S) cPent | | 0,42<br>C/EE 1:1 |
| LVII | 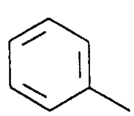 | (R&S) cPent | 132 | |

| Bsp.-Nr. | A | $R^1$ | Fp. (°C) | $R_f$ |
|---|---|---|---|---|
| LVIII | 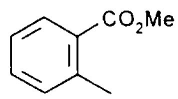 | (R&S) cPent | 183 | |
| LIX | 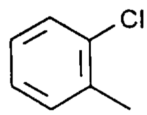 | (R&S) cPent | 134 | |
| LX | 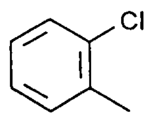 | (R&S) cHept | | 0,30 C/EE 7:3 |
| LXI | 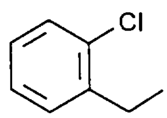 | (R&S) cPent | | 0,5 C/EE 1:1 |
| LXII | 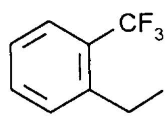 | (R&S) cPent | | 0,39 C/EE 1:1 |
| LXIII | 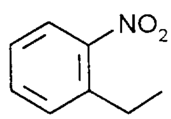 | (R&S) cPent | | 0,29 C/EE 1:1 |

**Tabelle III:**

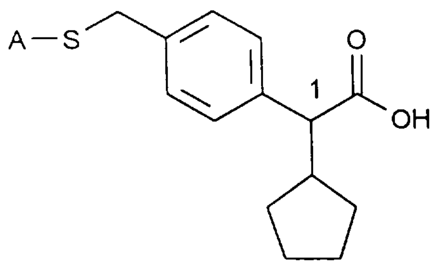

| Bsp.-Nr. | A | 1 | Fp. (°C) | $R_f$ |
|---|---|---|---|---|
| LXIV | 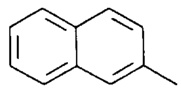 | R&S | | |
| LXV | 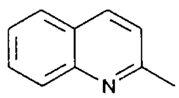 | R&S | | |
| LXVI | 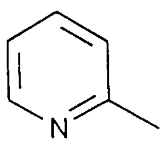 | R&S | | |
| LXVII | 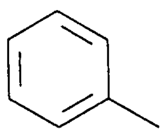 | R&S | | |
| LXVIII | 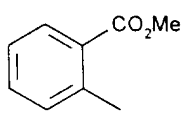 | R&S | | |

## Herstellungsbeispiele

### Beispiel 1

N-(2-Chlorbenzoyl)-2-cyclopentyl-4-(phenylamino-methyl)-phenyl-[(N'-(2-hydroxy)-1-(R)-phenvlethyl)]-2-essigsäure-amid

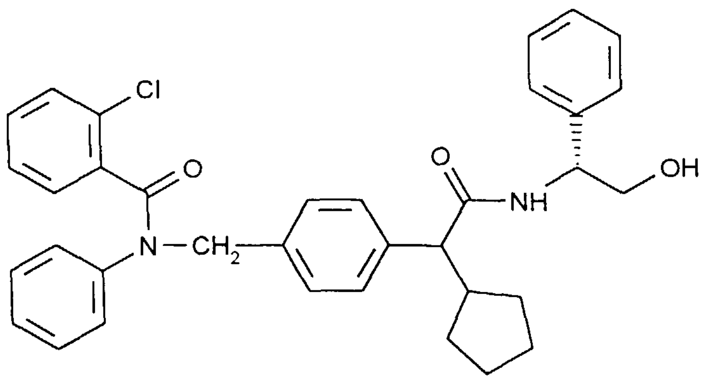

1,34 g (3 mmol) der Verbindung aus Beispiel II werden mit 0,412 g (3 mmol) R-(-)-2-Phenylglycinol (Aldrich) in 30 ml $CH_2Cl_2$ gelöst, anschließend werden 0,446 mg (3,3 mmol) 1-Hydroxy-1H-benzotriazol Hydrat (Aldrich) zugegeben. Nach Zugabe von 662 mg (3,45 mmol) N'-(3-Dimethylaminopropyl)-N-ethylcarbodiimid-hydrochlorid (Aldrich) und 0,8 ml Triethylamin wird über Nacht bei RT gerührt. Es wird mit $CH_2Cl_2$ verdünnt, je einmal mit $NH_4Cl$-Lösung und $NaHCO_3$-Lösung gewaschen, getrocknet und einrotiert. Es wird mit Cyclohexan / Essigester (1:1) chromatographiert.
Ausbeute: 1,67 g (98%).
$R_f$ = 0,17 (Cyclohexan / Essigester = 1:1)

In Analogie zur Vorschrift des Beispiels 1 werden die in den Tabellen 1 - 3 aufgeführten Verbindungen über die entsprechenden Vorstufen (analog der Beispiele I und II) hergestellt:

**Tabelle 1:**

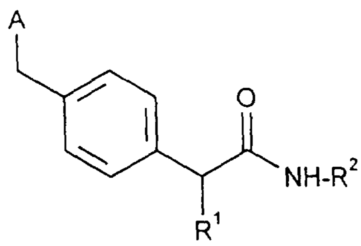

| Bsp.-Nr. | A | $R^1$ | $R^2$ | Fp. (°C) | $R_f$ |
|---|---|---|---|---|---|
| 2 | 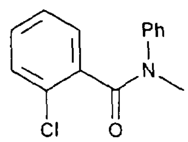 | (R&S) cPent | Bn | 176-177 | |
| 3 | 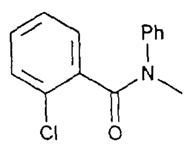 | (R&S) cHept | R-Gly | | 0,23 C/EE 1:1 |
| 4 | 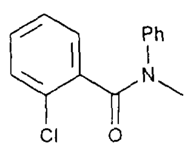 | (R&S) cHept | Bn | 171-172 | |
| 5 | 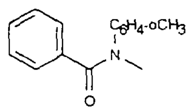 | (R&S) cPent | R-Gly | | 0,21 C/EE 1:1 |
| 6 | 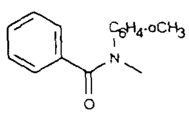 | (R&S) cPent | Bn | 182-183 | |
| 7 | 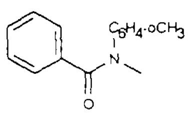 | (R&S) cHept | R-Gly | | 0,26 C/EE 1:1 |

| Bsp.-Nr. | A | $R^1$ | $R^2$ | Fp. (°C) | $R_f$ |
|---|---|---|---|---|---|
| 8 | 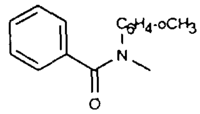 | (R&S) cHept | Bn | 146-147 | |
| 9 | 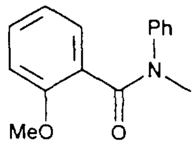 | (R&S) cPent | R-Gly | | 0,13 C/EE 1:1 |
| 10 | 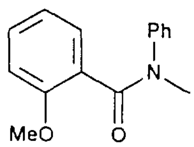 | (R&S) cPent | Bn | 171 | |
| 11 | 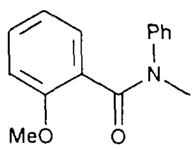 | (R&S) cHept | R-Gly | | 0,13 C/EE 1:1 |
| 12 | 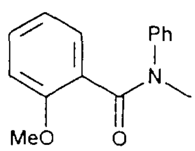 | (R&S) cHept | Bn | 144 | |
| 13 | 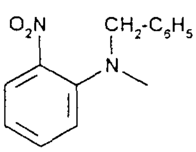 | (R&S) cPent | R-Gly | | 0,14 C/EE 1:1 |
| 14 | 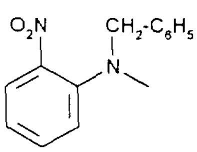 | (R&S) cPent | Bn | | 0,39 C/EE 1:1 |

| Bsp.-Nr. | A | $R^1$ | $R^2$ | Fp. (°C) | $R_f$ |
|---|---|---|---|---|---|
| 15 | 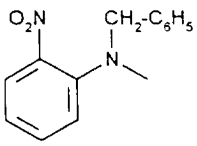 | (R&S) cHept | R-Gly | | 0,17 C/EE 1:1 |
| 16 | 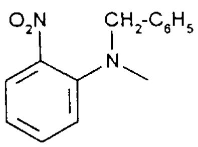 | (R&S) cHept | Bn | | 0,44 C/EE 1:1 |
| 17 | 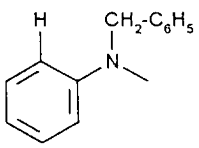 | (R&S) cPent | R-Gly | | 0,18 C/EE 1:1 |
| 18 | 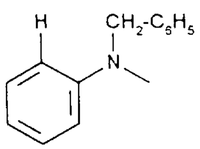 | (R&S) cPent | Bn | 160 | |
| 19 | 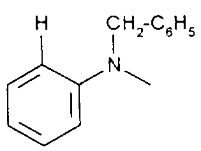 | (R&S) cHept | R-Gly | | 0,27 C/EE 1:1 |
| 20 | 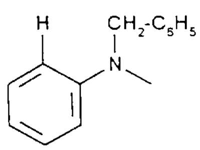 | (R&S) cHept | Bn | 166 | |
| 21 | 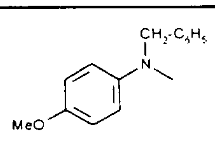 | (R&S) cPent | R-Gly | | 0,16 C/EE 1:1 |

| Bsp.-Nr. | A | $R^1$ | $R^2$ | Fp. (°C) | $R_f$ |
|---|---|---|---|---|---|
| 22 | 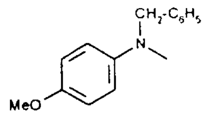 | (R&S) cPent | Bn | 159 | |
| 23 | 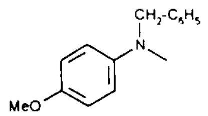 | (R&S) cHept | R-Gly | | 0,22 C/EE 1:1 |
| 24 | 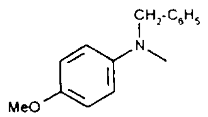 | (R&S) cHept | Bn | 171 | |
| 25 | 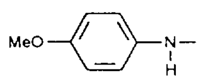 | (R&S) cPent | R-Gly | | 0,21 C/EE 1:1 |
| 26 | 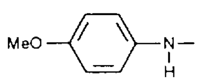 | (R&S) cPent | Bn | 146-147 | |
| 27 | 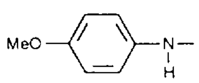 | (R&S) cHept | R-Gly | | 0,32 C/EE 1:1 |
| 28 | 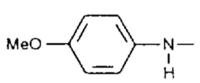 | (R&S) cHept | Bn | 105 | |
| 29 | 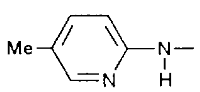 | (R&S) cPent | R-Gly | | 0,31 C/EE 4:6 |
| 30 | 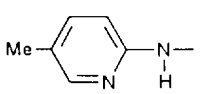 | (R&S) cPent | Bn | 133 | |
| 31 | 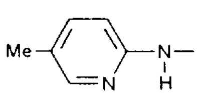 | (R&S) cHept | R-Gly | | 0,1 C/EE 1:1 |
| 32 | 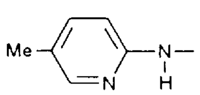 | (R&S) cHept | Bn | 110-111 | |

| Bsp.-Nr. | A | $R^1$ | $R^2$ | Fp. (°C) | $R_f$ |
|---|---|---|---|---|---|
| 33 | 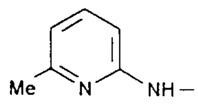 | (R&S) cPent | R-Gly | | 0,39 C/EE 4:6 |
| 34 | 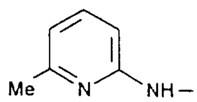 | (R&S) cPent | Bn | 159-160 | |
| 35 | 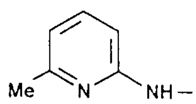 | (R&S) cHept | R-Gly | | 0,17 C/EE 4:6 |
| 36 | 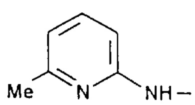 | (R&S) cHept | Bn | 151-152 | |
| 37 | 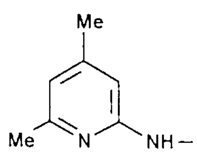 | (R&S) cPent | R-Gly | | 0,08 C/EE 1:1 |
| 38 | 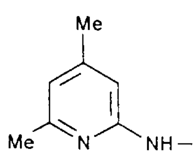 | (R&S) cPent | Bn | 161-162 | |
| 39 | 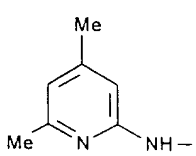 | (R&S) cHept | R-Gly | 129-130 | |
| 40 | 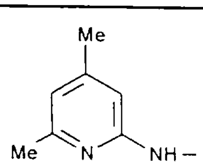 | (R&S) cHept | Bn | 155-156 | |

| Bsp.-Nr. | A | $R^1$ | $R^2$ | Fp. (°C) | $R_f$ |
|---|---|---|---|---|---|
| 41 | 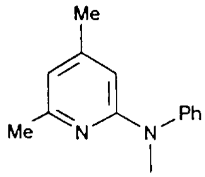 | (dia A) cPent | R-Gly | 177 | |
| 42 | 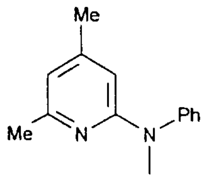 | (dia B) cPent | Bn | | 0,40 C/EE 1:1 |
| 43 | 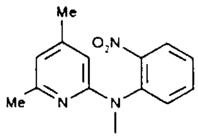 | (R&S) cPent | R-Gly | | 0,23 C/EE 7:3 |
| 44 | 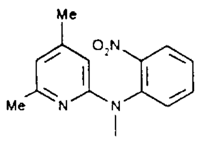 | (R&S) cPent | Bn | | 0,20 C/EE 7:3 |
| 45 | 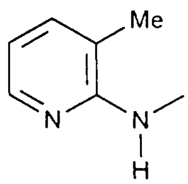 | (R&S) cPent | R-Gly | | 0,15 C/EE 1:1 |
| 46 | 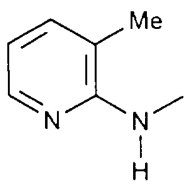 | (R&S) cPent | Bn | | 0,23 C/EE 7:3 |

| Bsp.-Nr. | A | $R^1$ | $R^2$ | Fp. (°C) | $R_f$ |
|---|---|---|---|---|---|
| 47 | 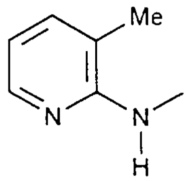 | (R&S) cHept | R-Gly | | 0,14 C/EE 1:1 |
| 48 | 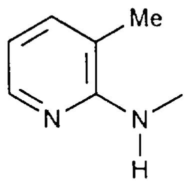 | (R&S) cHept | Bn | | 0,23 C/EE 7:3 |
| 49 | 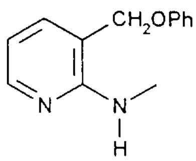 | (R&S) cPent | R-Gly | 134-135 | |
| 50 | 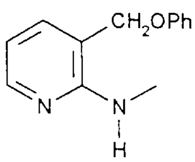 | (R&S) cPent | Bn | | 0,23 C/EE 7:3 |
| 51 | 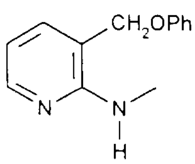 | (R&S) cHept | R-Gly | | 0,25 C/EE 1:1 |
| 52 | 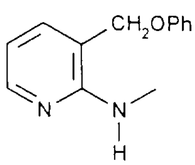 | (R&S) cHept | Bn | | 0,31 C/EE 7:3 |

| Bsp.-Nr. | A | $R^1$ | $R^2$ | Fp. (°C) | $R_f$ |
|---|---|---|---|---|---|
| 53 | 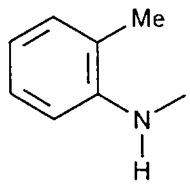 | (R&S) cPent | R-Gly | | 0,37 C/EE 1:1 |
| 54 | 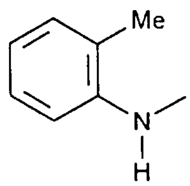 | (R&S) cPent | Bn | | 0,45 C/EE 7:3 |
| 55 | 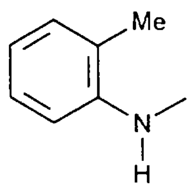 | (R&S) cHept | R-Gly | | 0,07 C/EE 7:3 |
| 56 | 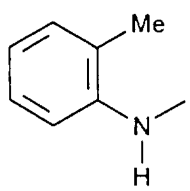 | (R&S) cHept | Bn | 149-150 | |
| 57 | 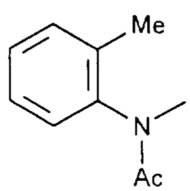 | (R&S) cHept | Bn | | 0,08 C/EE 4:6 |

| Bsp.-Nr. | A | $R^1$ | $R^2$ | Fp. (°C) | $R_f$ |
|---|---|---|---|---|---|
| 58 | 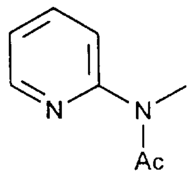 | (R&S) cPent | R-Gly | | 0,05 C/EE 4:6 |
| 59 | 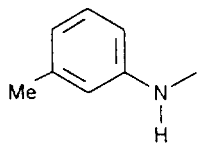 | (R&S) cPent | R-Gly | | 0,34 C/EE 1:1 |
| 60 | 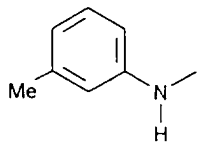 | (R&S) cPent | Bn | | 0,39 C/EE 7:3 |
| 61 | 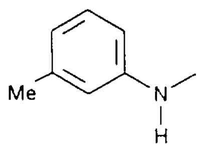 | (R&S) cHept | R-Gly | | 0,13 C/EE 7:3 |
| 62 | 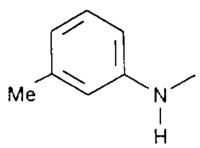 | (R&S) cHept | Bn | | 0,33 C/EE 7:3 |
| 63 | 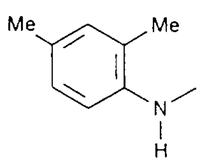 | (R&S) cPent | R-Gly | | 0,37 C/EE 1:1 |

| Bsp.-Nr. | A | $R^1$ | $R^2$ | Fp. (°C) | $R_f$ |
|---|---|---|---|---|---|
| 64 | 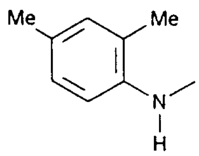 | (R&S) cPent | Bn | | 0,25 C/EE 7:3 |
| 65 | 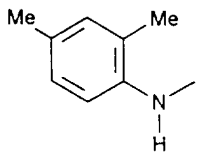 | (R&S) cHept | R-Gly | | 0,45 C//EE 1:1 |
| 66 | 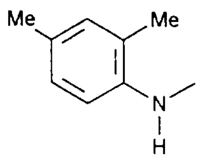 | (R&S) cHept | Bn | | 0,3 C/EE 7:3 |

C/EE = Cyclohexan : Essigester

**Tabelle 2:**

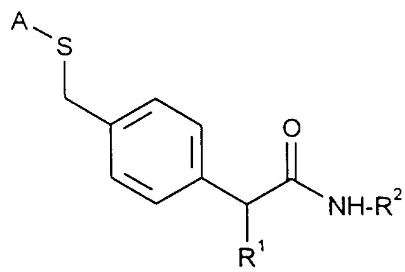

| Bsp.-Nr. | A | $R^1$ | $R^2$ | Fp. (°C) | $R_f$ |
|---|---|---|---|---|---|
| 67 | 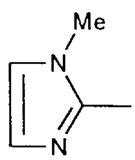 | (R&S) cPent | R-Gly | | 0,05 C/EE 3:7 |
| 68 | 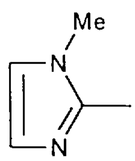 | (R&S) cPent | Bn | | 0,08 C/EE 1:1 |
| 69 | 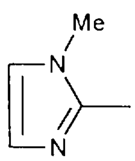 | (R&S) cHept | R-Gly | 155-156 | |
| 70 | 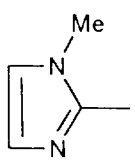 | (R&S) cHept | Bn | | 0,09 C/EE 1:1 |
| 71 | 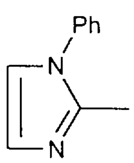 | (R&S) cPent | R-Gly | | 0,09 C/EE 1:1 |

| Bsp.-Nr. | A | $R^1$ | $R^2$ | Fp. (°C) | $R_f$ |
|---|---|---|---|---|---|
| 72 | 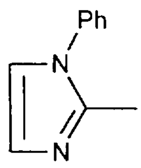 | (R&S) cPent | Bn | 108-109 | |
| 73 | 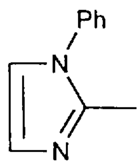 | (R&S) cHept | R-Gly | | 0,12 C/EE 1:1 |
| 74 | 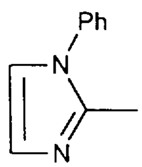 | (R&S) cHept | Bn | 144-145 | |
| 75 | 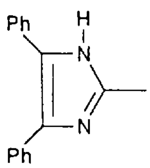 | (R&S) cPent | R-Gly | | 0,22 C/EE 1:1 |
| 76 | 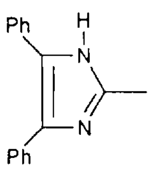 | (R&S) cPent | Bn | 140-141 | |
| 77 | 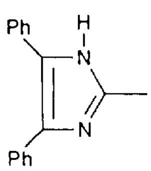 | (R&S) cHept | R-Gly | | 0,28 C/EE 1:1 |

| Bsp.-Nr. | A | $R^1$ | $R^2$ | Fp. (°C) | $R_f$ |
|---|---|---|---|---|---|
| 78 | 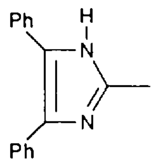 | (R&S) cHept | Bn | 186-187 | |
| 79 | 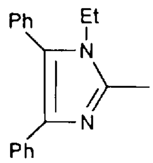 | (R&S) cPent | R-Gly | | 0,25 C/EE 1:1 |
| 80 | 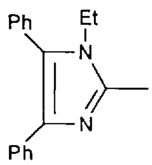 | (R&S) cPent | Bn | 135-136 | |
| 81 | 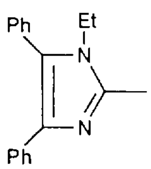 | (R&S) cHept | R-Gly | | 0,33 C/EE 1:1 |
| 82 | 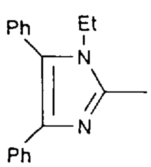 | (R&S) cHept | Bn | 173-174 | |
| 83 | 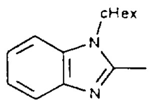 | (R&S) cPent | R-Gly | | 0,26 C/EE 1:1 |
| 84 | 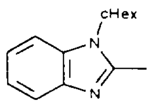 | (R&S) cPent | Bn | | 0,28 C/EE 7:3 |

| Bsp.-Nr. | A | $R^1$ | $R^2$ | Fp. (°C) | $R_f$ |
|---|---|---|---|---|---|
| 85 | 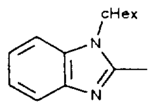 | (R&S) cHept | R-Gly | | 0,37 C/EE 1:1 |
| 86 | 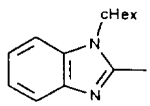 | (R&S) cHept | Bn | | 0,39 C/EE 7:3 |
| 87 | 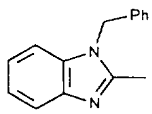 | (R&S) cPent | R-Gly | | 0,29 C/EE 1:1 |
| 88 | 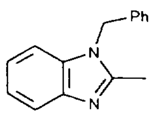 | (R&S) cPent | Bn | 138-139 | |
| 89 | 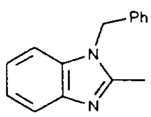 | (R&S) cHept | R-Gly | | 0,35 C/EE 1:1 |
| 90 | 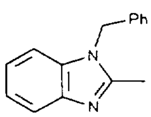 | (R&S) cHept | Bn | 164-165 | |
| 91 | 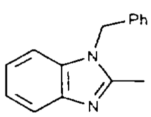 | (R&S) cPent | R-Gly | 181-182 | |
| 92 | 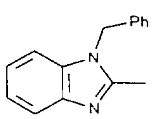 | (R&S) cPent | Bn | 154-155 | |
| 93 | 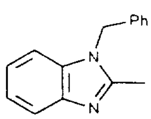 | (R&S) cHept | R-Gly | | 0,32 C/EE 1:1 |

| Bsp.-Nr. | A | $R^1$ | $R^2$ | Fp. (°C) | $R_f$ |
|---|---|---|---|---|---|
| 94 | 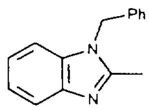 | (R&S) cHept | Bn | 158-159 | |
| 95 | 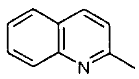 | (R&S) cPent | R-Gly | | 0,25 C/EE 1:1 |
| 96 | 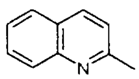 | (dia A) cPent | R-Gly | 188 | |
| 97 | 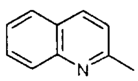 | (dia B) cPent | R-Gly | 143 | |
| 98 | 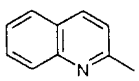 | (R&S) cHept | R-Gly | 139-140 | |
| 99 | 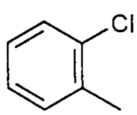 | (R&S) cPent | Bn | | 0,43 C/EE 7:3 |
| 100 | 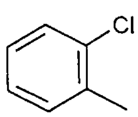 | (R&S) cHept | Bn | | 0,46 C/EE 7:3 |
| 101 | 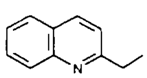 | (R&S) cPent | R-Gly | | 0,33 C/EE 1:1 |
| 102 | 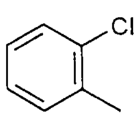 | (R&S) cPent | R-Gly | 150-151 | |
| 103 | 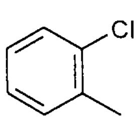 | (R&S) cPent | R-Gly | 155-156 | |

| Bsp.-Nr. | A | $R^1$ | $R^2$ | Fp. (°C) | $R_f$ |
|---|---|---|---|---|---|
| 104 | 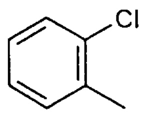 | (R&S) cPent | R-Gly | 167-168 | |
| 105 | 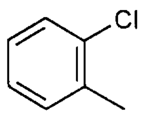 | (R&S) cPent | R-Gly | 174-175 | |
| 106 | 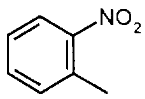 | (R&S) cPent | R-Gly | 158-160 | |
| 107 | 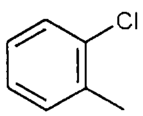 | (R&S) cHept | R-Gly | 156-157 | |
| 108 | 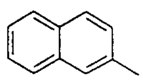 | (dia A) cPent | R-Gly | 164 | |
| 109 | 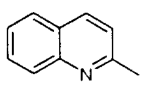 | (dia B) cPent | R-Gly | | 0,32 C/EE 1:1 |
| 110 | 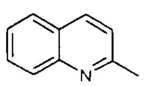 | (dia A) cPent | R-Gly | 178 | |
| 111 | 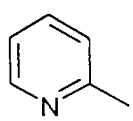 | (dia B) cPent | R-Gly | | 0,32 C/EE 1:1 |
| 112 | 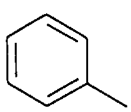 | (R&S) cPent | R-Gly | 150 | |

Tabelle 3:

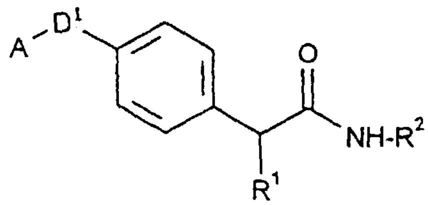

| Bsp.-Nr. | A | $D^1$ | $R^1$ | $R^2$ | Fp. (°C) | $R_f$ |
|---|---|---|---|---|---|---|
| 119 | 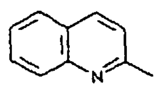 | O-$CH_2$ | (R&S) cPent | R-Gly | 155 | |

**Tabelle 4:**

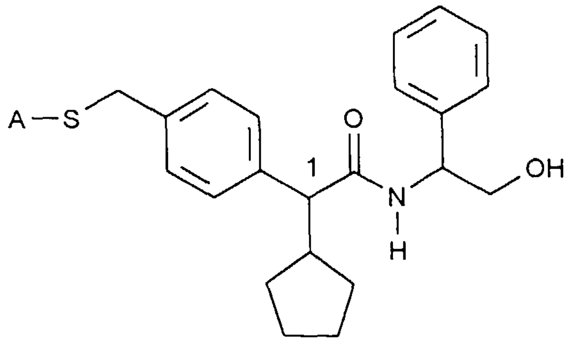

| Bsp.-Nr. | A | 1 | Fp. (°C) | $R_f$ |
|---|---|---|---|---|
| 130 | 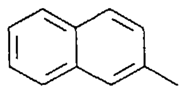 | R&S | | 0,14 C/EE 4:6 |
| 131 | 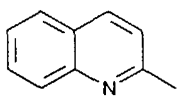 | dia A | 137-139 | |
| 132 | 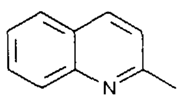 | dia B | | 0,08 C/EE 1:1 |
| 133 | 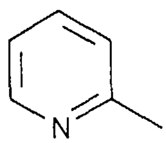 | R&S | | 0,06 C/EE 4:6 |
| 134 | 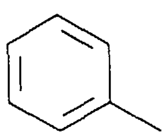 | R&S | 166-167 | |
| 135 | 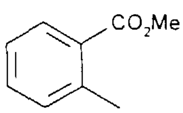 | R&S | | 0,09 C/EE 4:6 |

## Patentansprüche

1. Heteroverknüpfte Phenylglycinolamide der allgemeinen Formel (I)

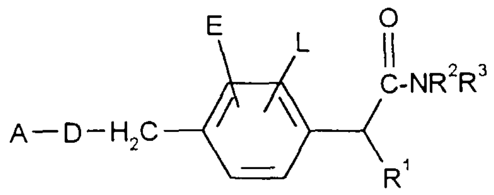

(I)

in welcher

A für Aryl mit 6 bis 10 Kohlenstoffatomen, Benzyl oder für einen, gegebenenfalls benzokondensierten 5- bis 7-gliedrigen gesättigten oder ungesättigten, partiell ungesättigten oder ungesättigten Heterocyclus mit bis zu 3 Heteroatomen aus der Reihe S, N und/oder O steht, wobei die Ringsysteme gegebenenfalls - auch über die N-Funktion - bis zu 3-fach gleich oder verschieden durch Halogen, Trifluormethyl, Carboxy, Hydroxyl, Nitro, Cycloalkyl mit 3 bis 7 Kohlenstoffatomen, Benzyl, Phenyl, Benzyloxy oder durch geradkettiges oder verzweigtes Alkyl, Alkoxy oder Alkoxycarbonyl mit jeweils bis zu 6 Kohlenstoffatomen substituiert sind, oder für einen Rest der Formel $R^5R^4N$- oder

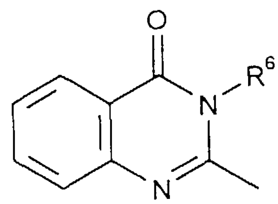

steht,
worin

$R^4$ und $R^5$ gleich oder verschieden sind und Wasserstoff, Phenyl oder geradkettiges oder verzweigtes Alkyl mit bis zu 6 Kohlenstoffatomen bedeuten,

$R^6$ Wasserstoff, Phenyl oder geradkettiges oder verzweigtes Alkyl mit bis zu 4 Kohlenstoffatomen bedeutet,

D für ein Sauerstoffatom oder für einen Rest der Formel -CO-, $-(CO)_a-NR^7$, $-(CH_2)_bS-$, $-(CH_2)_c-NR^8$ oder -CH=CH- steht,
worin

a, b und c gleich oder verschieden sind und eine Zahl 0 oder 1 bedeuten,

$R^7$ und $R^8$ gleich oder verschieden sind und Wasserstoff, geradkettiges oder verzweigtes Alkyl oder Acyl mit jeweils bis zu 6 Kohlenstoffatomen, Phenyl oder Benzyl bedeuten, wobei die Ringsysteme gegebenenfalls bis zu 2-fach gleich oder verschieden durch Nitro, Halogen, Trifluormethyl oder durch geradkettiges oder verzweigtes Alkyl oder Alkoxy mit jeweils bis zu 3 Kohlenstoffatomen substituiert sind,

E und L gleich oder verschieden sind und für Wasserstoff, Cycloalkyl mit 3 bis 8 Kohlenstoffatomen, Azido, Hydroxy, Halogen, geradkettiges oder verzweigtes Alkyl, Alkoxy oder Alkenyl mit jeweils bis zu 6 Kohlenstoffatomen stehen,

$R^1$ für Cycloalkyl mit 3 bis 8 Kohlenstoffatomen steht, oder für geradketiges oder verzweigtes Alkyl mit

bis zu 10 Kohlenstoffatomen steht,

$R^2$ für Wasserstoff oder für geradkettiges oder verzweigtes Alkyl mit bis zu 4 Kohlenstoffatomen steht,

$R^3$ für einen Rest der Formel

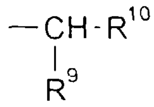

steht,
worin

$R^9$ Wasserstoff oder einen Rest der Formel $CH_2$-OH bedeutet,

$R^{10}$ Phenyl bedeutet, das gegebenenfalls bis zu 3-fach gleich oder verschieden durch Hydroxy, Halogen oder geradkettiges oder verzweigtes Alkyl mit bis zu 5 Kohlenstoffatomen substituiert ist,

und deren Salze.

**2.** Heteroverknüpfte Phenylglycinolamide der Formel nach Anspruch 1, in welcher

A für Naphthyl, Phenyl, Benzyl, Pyridyl, Imidazolyl, Benzimidazolyl oder Chinolyl steht, die gegebenenfalls - auch über die N-Funktion - bis zu 3-fach gleich oder verschieden durch Fluor, Chlor, Brom, Trifluormethyl, Carboxy, Hydroxyl, Nitro, Cyclopropyl, Cylcopentyl, Cyclohexyl, Benzyl, Phenyl, Benzyloxy oder durch geradkettiges oder verzweigtes Alkyl, Alkoxy oder Alkoxycarbonyl mit jeweils bis zu 4 Kohlenstoffatomen substituiert sind, oder für einen Rest der Formel $R^5R^4N$- oder

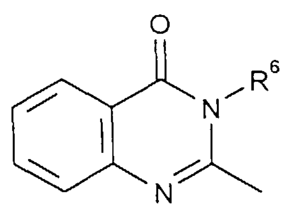

steht,
worin

$R^4$ und $R^5$ gleich oder verschieden sind und Wasserstoff, Phenyl oder geradkettiges oder verzweigtes Alkyl mit bis zu 4 Kohlenstoffatomen bedeuten,

$R^6$ Wasserstoff, Phenyl oder geradkettiges oder verzweigtes Alkyl mit bis zu 3 Kohlenstoffatomen bedeutet,

D für ein Sauerstoffatom oder für einen Rest der Formel -CO-, $-(CO)_a-NR^7$, $-(CH_2)_bS-$, $-(CH_2)_c-NR^8$ oder -CH=CH- steht,
worin

a, b und c gleich oder verschieden sind und eine Zahl 0 oder 1 bedeuten,

$R^7$und $R^8$ gleich oder verschieden sind und Wasserstoff, geradkettiges oder verzweigtes Alkyl oder Acyl mit jeweils bis zu 5 Kohlenstoffatomen, Phenyl oder Benzyl bedeuten, wobei die Ringsysteme gegebenenfalls bis zu 2-fach gleich oder verschieden durch Nitro, Fluor, Chlor, Brom, Trifluormethyl oder durch geradkettiges oder verzweigtes Alkyl

oder Alkoxy mit jeweils bis zu 3 Kohlenstoffatomen substituiert sind,

E und L gleich oder verschieden sind und für Wasserstoff, Cyclopropyl, Cyclopentyl, Cyclohexyl, Azido, Hydroxy, Fluor, Chlor, Brom, geradkettiges oder verzweigtes Alkyl, Alkoxy oder Alkenyl mit jeweils bis zu 3 Kohlenstoffatomen stehen,

$R^1$ für Cyclopropyl, Cylcobutyl, Cyclopentyl, Cyclohexyl oder Cycloheptyl steht, oder für geradkettiges oder verzweigtes Alkyl mit bis zu 8 Kohlenstoffatomen steht,

$R^2$ für Wasserstoff oder für geradkettiges oder verzweigtes Alkyl mit bis zu 3 Kohlenstoffatomen steht,

$R^3$ für einen Rest der Formel

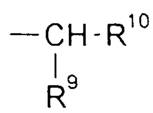

steht,
worin

$R^9$ Wasserstoff oder einen Rest der Formel $CH_2$-OH bedeutet,

$R^{10}$ Phenyl bedeutet, das gegebenenfalls bis zu 2-fach gleich oder verschieden durch Hydroxy, Fluor, Chlor, Brom oder geradkettiges oder verzweigtes Alkyl mit bis zu 3 Kohlenstoffatomen substituiert ist,

und deren Salze.

**3.** Heteroverknüpfte Phenylglycinolamide der Formel nach Anspruch 1, in welcher

A für Naphthyl, Phenyl, Benzyl, Pyridyl, Imidazolyl, Benzimidazolyl oder Chinolyl steht, die gegebenenfalls - auch über die N-Funktion - bis zu 2-fach gleich oder verschieden durch Fluor, Chlor, Trifluormethyl, Carboxy, Hydroxyl, Nitro, Cyclohexyl, Benzyl, Phenyl, Benzyloxy oder durch geradkettiges oder verzweigtes Alkyl, Alkoxy oder Alkoxycarbonyl mit jeweils bis zu 3 Kohlenstoffatomen substituiert sind, oder
für einen Rest der Formel $R^5R^4N$- oder

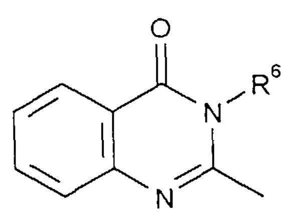

steht,
worin

$R^4$ und $R^5$ gleich oder verschieden sind und Wasserstoff, Phenyl oder Methyl bedeuten,

$R^6$ Wasserstoff, Phenyl oder Methyl bedeutet,

D für ein Sauerstoffatom oder für einen Rest der Formel -CO-, $-(CO)_a-NR^7$, $-(CH_2)_bS-$, $-(CH_2)_c-NR^8$ oder -CH=CH- steht,
worin

a, b und c gleich oder verschieden sind und eine Zahl 0 oder 1 bedeuten,

$R^7$ und $R^8$ gleich oder verschieden sind und Wasserstoff, Methyl, Ethyl, Phenyl, Acetyl oder Benzyl bedeuten, wobei die Ringsysteme gegebenenfalls bis zu 2-fach gleich oder verschieden durch Nitro, Fluor, Chlor, Brom, Trifluormethyl oder durch geradkettiges oder verzweigtes Alkyl oder Alkoxy mit jeweils bis zu 3 Kohlenstoffatomen substituiert sind,

E und L gleich oder verschieden sind und für Wasserstoff, Fluor, Chlor oder Brom stehen,

$R^1$ für Cyclopropyl, Cylcobutyl, Cyclopentyl oder Cyclohexyl steht, oder für geradkettiges oder verzweigtes Alkyl mit bis zu 8 Kohlenstoffatomen steht,

$R^2$ für Wasserstoff oder für geradkettiges oder verzweigtes Alkyl mit bis zu 3 Kohlenstoffatomen steht,

$R^3$ für einen Rest der Formel

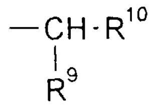

steht,
worin

$R^9$ Wasserstoff oder einen Rest der Formel $CH_2$-OH bedeutet,

$R^{10}$ Phenyl bedeutet, das gegebenenfalls bis zu 2-fach gleich oder verschieden durch Hydroxy, Fluor, Chlor, Methyl oder Ethyl substituiert ist,

und deren Salze.

**4.** Heteroverknüpfte Phenylglycinolamide nach Anspruch 1 bis 3 als Arzneimittel.

**5.** Verfahren zur Herstellung von heteroverknüpften Phenylglycinolamiden nach Anspruch 1 bis 3, dadurch gekennzeichnet, daß man Carbonsäuren der allgemeinen Formel (II),

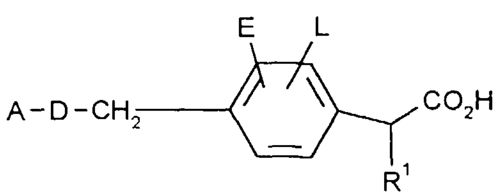

(II)

in welcher

A, D, E, L und $R^1$ die oben angegebene Bedeutung haben,
gegebenenfalls unter vorgeschalteter Aktivierung der Carbonsäurefunktion, mit Phenylglycinolen der allgemeinen Formel (III)

$$HR^2N\text{-}R^3 \quad (III)$$

in welcher
$R^2$ und $R^3$ die oben angegebene Bedeutung haben,

gegebenenfalls unter Schutzgasatmosphäre, gegebenenfalls in inerten Lösemitteln, in Anwesenheit einer Base und/oder Hilfsmittels umsetzt.

**6.** Arzneimittel enthaltend mindestens ein heteroverknüpftes Phenylglycinolamid nach Anspruch 1 bis 3 und ein pharmakologisch unbedenkliches Formulierungshilfsmittel.

**7.** Arzneimittel nach Anspruch 6 zur Behandlung von Atherosklerose.

**8.** Verwendung von heteroverknüpften Phenylglycinolamiden nach Anspruch 1 bis 3 zur Herstellung von Arzneimitteln.

**9.** Verwendung nach Anspruch 8 zur Herstellung von antiatherosklerotisch wirksamen Arzneimitteln.

**10.** Verwendung von heteroverknüpften Phenylglycinolamiden nach Anspruch 1 bis 3 zur Herstellung von Arzneimitteln zur Verminderung oder vollständigen Inhibierung der Bildung und/oder der Freisetzung von Apo B-100-assoziierten Lipoproteinen.

**11.** Verwendung nach Anspruch 8 zur Herstellung von Arzneimitteln zur Prävention und Behandlung von Adipositas.

**Revendications**

**1.** Phénylglycinolamides à liaison hétéroatomique de formule générale (I)

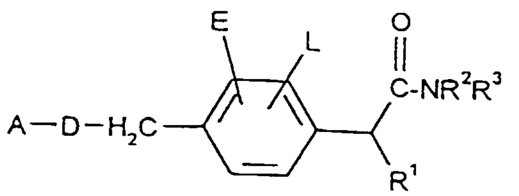

(I)

dans laquelle

A désigne un groupe aryle ayant 6 à 10 atomes de carbone, un groupe benzyle ou un hétérocycle pentagonal à heptagonal saturé ou insaturé, partiellement insaturé ou insaturé, éventuellement condensé au benzène, ayant jusqu'à 3 hétéroatomes de la série S, N et/ou O, les systèmes cycliques étant éventuellement substitués - également par l'intermédiaire de la fonction N - jusqu'à 3 fois identiques ou différentes par un halogène, un radical trifluorométhyle, carboxy, hydroxyle, nitro, cycloalkyle ayant 3 à 7 atomes de carbone, benzyle, phényle, benzyloxy ou par un radical alkyle, alkoxy ou alkoxycarbonyle linéaire ou ramifié ayant dans chaque cas jusqu'à 6 atomes de carbone, ou bien un reste de formule $R^5R^4N$- ou

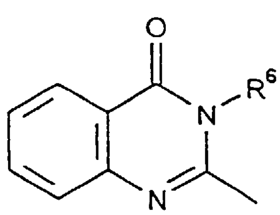

où

$R^4$ et $R^5$ sont identiques ou différents et représentent l'hydrogène, un groupe phényle ou un groupe alkyle linéaire ou ramifié ayant jusqu'à 6 atomes de carbone,

$R^6$ représente l'hydrogène, un groupe phényle ou un groupe alkyle linéaire ou ramifié ayant

jusqu'à 4 atomes de carbone,

D représente un atome d'oxygène ou un reste de formule -CO-, $-(CO)_a-NR^7$, $-(CH_2)_bS-$, $-(CH_2)_c-NR^8$ ou -CH=CH-,
où

a, b et c sont égaux ou différents et représentent le nombre 0 ou 1,
$R^7$ et $R^8$ sont identiques ou différents et représentent l'hydrogène, un groupe alkyle ou acyle linéaire ou ramifié ayant chacun jusqu'à 6 atomes de carbone, phényle ou benzyle, les systèmes cycliques étant éventuellement substitués jusqu'à 2 fois identiques ou différentes par un radical nitro, halogéno, trifluorométhyle ou par un radical alkyle ou alkoxy linéaire ou ramifié ayant chacun jusqu'à 3 atomes de carbone,

E et L sont identiques ou différents et représentent l'hydrogène, un groupe cycloalkyle ayant 3 à 8 atomes de carbone, azido, hydroxy, un halogène, un groupe alkyle, alkoxy ou alcényle linéaire ou ramifié ayant dans chaque cas jusqu'à 6 atomes de carbone,
$R^1$ est un groupe cycloalkyle ayant 3 à 8 atomes de carbone ou un groupe alkyle linéaire ou ramifié ayant jusqu'à 10 atomes de carbone,
$R^2$ représente l'hydrogène ou un groupe alkyle linéaire ou ramifié ayant jusqu'à 4 atomes de carbone,
$R^3$ est un reste de formule

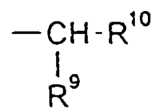

où

$R^9$ représente l'hydrogène ou un reste de formule $CH_2$-OH,
$R^{10}$ représente un groupe phényle qui est substitué, le cas échéant, jusqu'à 3 fois identiques ou différentes par un radical hydroxy, halogéno ou alkyle linéaire ou ramifié ayant jusqu'à 5 atomes de carbone,

et leurs sels.

**2.** Phénylglycinolamides à liaison hétéro-atomique de formule suivant la revendication 1, dans laquelle

A désigne un groupe naphtyle, phényle, benzyle, pyridyle, imidazolyle, benzimidazolyle ou quinolyle, ces groupes étant substitués, le cas échéant - également par l'intermédiaire de la fonction N - jusqu'à 3 fois identiques ou différentes par du fluor, du chlore, du brome, un radical trifluorométhyle, carboxy, hydroxyle, nitro, cyclopropyle, cyclopentyle, cyclohexyle, benzyle, phényle, benzyloxy ou par un radical alkyle, alkoxy ou alkoxycarbonyle linéaire ou ramifié ayant dans chaque cas jusqu'à 4 atomes de carbone, ou bien
un reste de formule $R^5R^4N$- ou

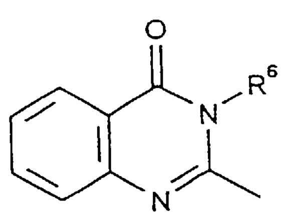

où

$R^4$ et $R^5$ sont identiques ou différents et représentent l'hydrogène, un groupe phényle ou un groupe alkyle linéaire ou ramifié ayant jusqu'à 4 atomes de carbone,

$R^6$ représente l'hydrogène, un groupe phényle ou un groupe alkyle linéaire ou ramifié ayant jusqu'à 3 atomes de carbone,

D représente un atome d'oxygène ou un reste de formule -CO-, $-(CO)_a-NR^7$, $-(CH_2)_bS-$, $-(CH_2)_c-NR^8$ ou -CH=CH-,
où

a, b et c sont identiques ou différents et représentent le nombre 0 ou 1,

$R^7$ et $R^8$ sont identiques ou différents et représentent l'hydrogène, un groupe alkyle ou acyle linéaire ou ramifié ayant chacun jusqu'à 5 atomes de carbone, un groupe phényle ou benzyle, les systèmes cycliques étant substitués, le cas échéant, jusqu'à 2 fois identiques ou différentes par un radical nitro, fluoro, chloro, bromo, trifluorométhyle ou par un radical alkyle ou alkoxy linéaire ou ramifié ayant dans chaque cas jusqu'à 3 atomes de carbone,

E et L sont identiques ou différents et représentent l'hydrogène, un groupe cyclopropyle, cyclopentyle, cyclohexyle, azido, hydroxy, du fluor, du chlore, du brome, un groupe alkyle, alkoxy ou alcényle linéaire ou ramifié ayant chacun jusqu'à 3 atomes de carbone,

$R^1$ est un groupe cyclopropyle, cyclobutyle, cyclopentyle, cyclohexyle ou cycloheptyle, ou bien un groupe alkyle linéaire ou ramifié ayant jusqu'à 8 atomes de carbone,

$R^2$ représente l'hydrogène ou un groupe alkyle linéaire ou ramifié ayant jusqu'à 3 atomes de carbone,

$R^3$ est un reste de formule

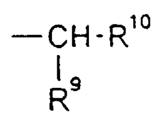

dans laquelle

$R^9$ désigne l'hydrogène ou un reste de formule $CH_2$-OH,

$R^{10}$ désigne un groupe phényle qui est substitué, le cas échéant, jusqu'à 2 fois identiques ou différentes par un radical hydroxy, fluoro, chloro, bromo ou un radical alkyle linéaire ou ramifié ayant jusqu'à 3 atomes de carbone,

et leurs sels.

**3.** Phénylglycinolamides à liaison hétéro-atomique de formule suivant la revendication 1,
dans laquelle

A désigne un groupe naphtyle, phényle, benzyle, pyridyle, imidazolyle, benzimidazolyle ou quinolyle, ces groupes étant substitués, le cas échéant - également par l'intermédiaire de la fonction N - jusqu'à 2 fois identiques ou différentes par du fluor, du chlore, un radical trifluorométhyle, carboxy, hydroxyle, nitro, cyclohexyle, benzyle, phényle, benzyloxy ou par un radical alkyle, alkoxy ou alkoxycarbonyle linéaire ou ramifié ayant dans chaque cas jusqu'à 3 atomes de carbone, ou bien un reste de formule $R^5R^4N$- ou

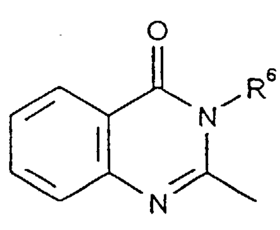

où

$R^4$ et $R^5$ sont identiques ou différents et représentent l'hydrogène, un groupe phényle ou méthyle,
$R^6$ représente l'hydrogène, un groupe phényle ou méthyle,

D représente un atome d'oxygène ou un reste de formule -CO-, $-(CO)_a-NR^7$,$-(CH_2)_bS-$, $-(CH_2)_c-NR^8$ ou -CH=CH-,
où

a, b et c sont identiques ou différents et représentent le nombre 0 ou 1,
$R^7$ et $R^8$ sont identiques ou différents et représentent l'hydrogène, un groupe méthyle, éthyle, phényle, acétyle ou benzyle, les systèmes cycliques étant éventuellement substitués jusqu'à 2 fois identiques ou différentes par un radical nitro, fluoro, chloro, bromo, trifluorométhyle ou par un radical alkyle ou alkoxy linéaire ou ramifié ayant jusqu'à 3 atomes de carbone,

E et L sont identiques ou différents et représentent l'hydrogène, le fluor, le chlore ou le brome,
$R^1$ est un groupe cyclopropyle, cyclobutyle, cyclopentyle ou cyclohexyle,
ou bien
un groupe alkyle linéaire ou ramifié ayant jusqu'à 8 atomes de carbone,
$R^2$ représente l'hydrogène ou un groupe alkyle linéaire ou ramifié ayant jusqu'à 3 atomes de carbone,
$R^3$ représente un reste de formule

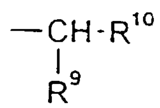

dans laquelle

$R^9$ représente l'hydrogène ou un reste de formule $CH_2$-OH,
$R^{10}$ désigne un groupe phényle qui est substitué, le cas échéant, jusqu'à 2 fois identiques ou différentes par un radical hydroxy, fluoro, chloro, méthyle ou éthyle,

et leurs sels.

**4.** Phénylglycinolamides à liaison hétéro-atomique suivant les revendications 1 à 3, utilisés comme médicaments.

**5.** Procédé de production de phénylglycinolamides à liaison hétéro-atomique suivant les revendications 1 à 3, caractérisé en ce qu'on fait réagir des acides carboxyliques de formule générale (II)

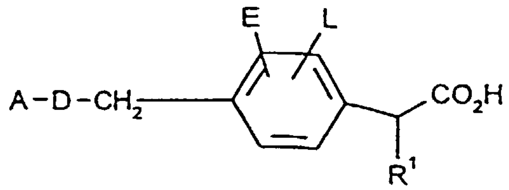

(II)

dans laquelle

A, D, E, L et $R^1$ ont la définition indiquée ci-dessus, le cas échéant avec activation préalable de la fonction acide carboxylique,
avec des phénylglycinols de formule générale (III)

$$HR^2\text{-}N\text{-}R^3 \quad (III)$$

dans laquelle

$R^2$ et $R^3$ ont la définition indiquée ci-dessus,

le cas échéant sous atmosphère de gaz protecteur, éventuellement dans des solvants inertes, en présence d'une base et/ou d'un agent auxiliaire.

**6.** Médicaments contenant au moins un phénylglycinolamide à liaison hétéroatomique suivant les revendications 1 à 3 et un auxiliaire de formulation acceptable du point de vue pharmacologique.

**7.** Médicaments suivant la revendication 6, destinés au traitement de l'athérosclérose.

**8.** Utilisation de phénylglycinolamides à liaison hétéro-atomique suivant les revendications 1 à 3 pour la préparation de médicaments.

**9.** Utilisation suivant la revendication 8, pour la préparation de médicaments à activité anti-athérosclérotique.

**10.** Utilisation de phénylglycinolamides à liaison hétéroatomique suivant les revendications 1 à 3 pour la préparation de médicaments destinés à atténuer ou à inhiber totalement la formation et/ou la libération de lipoprotéines associées à l'Apo B-100.

**11.** Utilisation suivant la revendication 8, pour la préparation de médicaments destinés à prévenir et à traiter l'adipositė.

## Claims

**1.** Hetero-linked phenylglycinolamides of the general formula (I)

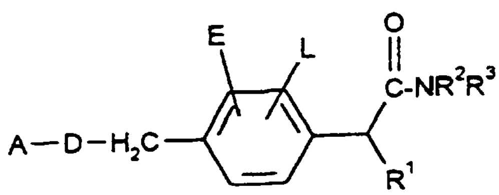

(I)

in which

A represents aryl having 6 to 10 carbon atoms, benzyl or an optionally benzo-fused 5- to 7-membered saturated, partially unsaturated or unsaturated heterocycle having up to 3 heteroatoms from the series S, N and/or O, the ring systems optionally being substituted - also via the N function - up to 3 times identically or differently by halogen, trifluoromethyl, carboxyl, hydroxyl, nitro, cycloalkyl having 3 to 7 carbon atoms, benzyl, phenyl, benzyloxy or by straight-chain or branched alkyl, alkoxy or alkoxycarbonyl each having up to 6 carbon atoms, or a radical of the formula $R^5R^4N$- or

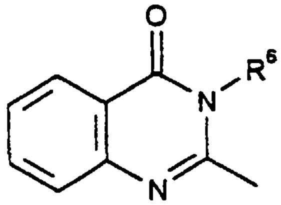

in which

$R^4$ and $R^5$ are identical or different and denote hydrogen, phenyl or straight-chain or branched alkyl having up to 6 carbon atoms,

$R^6$ denotes hydrogen, phenyl or straight-chain or branched alkyl having up to 4 carbon atoms,

D represents an oxygen atom or a radical of the formula -CO-, $-(CO)_a-NR^7$, $-(CH_2)_bS-$, $-(CH_2)_c-NR^8$ or -CH=CH-,
in which

a, b and c are identical or different and denote a number 0 or 1,

$R^7$ and $R^8$ are identical or different and denote hydrogen, straight-chain or branched alkyl or acyl each having up to 6 carbon atoms, phenyl or benzyl, the ring systems optionally being substituted up to 2 times identically or differently by nitro, halogen, trifluoromethyl or by straight-chain or branched alkyl or alkoxy each having up to 3 carbon atoms,

E and L are identical or different and represent hydrogen, cycloalkyl having 3 to 8 carbon atoms, azido, hydroxyl, halogen, straight-chain or branched alkyl, alkoxy or alkenyl each having up to 6 carbon atoms,

$R^1$ represents cycloalkyl having 3 to 8 carbon atoms, or represents straight-chain or branched alkyl having up to 10 carbon atoms,

$R^2$ represents hydrogen or straight-chain or branched alkyl having up to 4 carbon atoms,

$R^3$ represents a radical of the formula

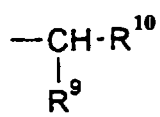

in which

$R^9$ denotes hydrogen or a radical of the formula $CH_2$-OH,

$R^{10}$ denotes phenyl which is optionally substituted up to 3 times identically or differently by hydroxyl, halogen or straight-chain or branched alkyl having up to 5 carbon atoms,

and their salts.

**2.** Hetero-linked phenylglycinolamides of the formula according to Claim 1, in which

A represents naphthyl, phenyl, benzyl, pyridyl, imidazolyl, benzimidazolyl or quinolyl, each of which is optionally substituted - also via the N function - up to 3 times identically or differently by fluorine, chlorine, bromine, trifluoromethyl, carboxyl, hydroxyl, nitro, cyclopropyl, cyclopentyl, cyclohexyl, benzyl, phenyl, benzyloxy or by straight-chain or branched alkyl, alkoxy or alkoxycarbonyl each having up to 4 carbon atoms, or
a radical of the formula $R^5R^4N$- or

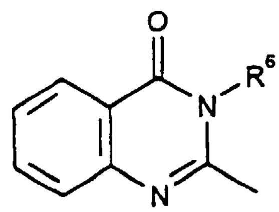

in which

$R^4$ and $R^5$ are identical or different and denote hydrogen, phenyl or straight-chain or branched alkyl having up to 4 carbon atoms,

$R^6$ denotes hydrogen, phenyl or straight-chain or branched alkyl having up to 3 carbon atoms,

D represents an oxygen atom or a radical of the formula -CO-, $-(CO)_a-NR^7$, $-(CH_2)_bS-$, $-(CH_2)_c-NR^8$ or -CH=CH-,
in which

a, b and c are identical or different and denote a number 0 or 1,

$R^7$ and $R^8$ are identical or different and denotes hydrogen, straight-chain or branched alkyl or acyl each having up to 5 carbon atoms, phenyl or benzyl, the ring systems optionally being substituted up to 2 times identically or differently by nitro, fluorine, chlorine, bromine, trifluoromethyl or by straight-chain or branched alkyl or alkoxy each having up to 3 carbon atoms,

E and L are identical or different and
represent hydrogen, cyclopropyl, cyclopentyl, cyclohexyl, azido, hydroxyl, fluorine, chlorine, bromine, straight-chain or branched alkyl, alkoxy or alkenyl each having up to 3 carbon atoms,

$R^1$ represents cyclopropyl, cyclobutyl, cyclopentyl, cyclohexyl or cycloheptyl, or
straight-chain or branched alkyl having up to 8 carbon atoms,

$R^2$ represents hydrogen or straight-chain or branched alkyl having up to 3 carbon atoms,

$R^3$ represents a radical of the formula,

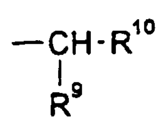

in which

$R^9$ denotes hydrogen or a radical of the formula $CH_2$-OH,

$R^{10}$ denotes phenyl which is optionally substituted up to 2 times identically or differently by hydroxyl, fluorine, chlorine, bromine or straight-chain or branched alkyl having up to 3 carbon atoms,

and their salts.

**3.** Hetero-linked phenylglycinolamides of the formula according to Claim 1, in which

A represents naphthyl, phenyl, benzyl, pyridyl, imidazolyl, benzimidazolyl or quinolyl, each of which is

optionally substituted - also via the N function - up to 2 times identically or differently by fluorine, chlorine, trifluoromethyl, carboxyl, hydroxyl, nitro, cyclohexyl, benzyl, phenyl, benzyloxy or by straight-chain or branched alkyl, alkoxy or alkoxycarbonyl each having up to 3 carbon atoms, or
a radical of the formula $R^5R^4N$- or

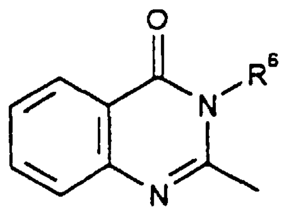

in which

$R^4$ and $R^5$ are identical or different and denote hydrogen, phenyl or methyl,

$R^6$ denotes hydrogen, phenyl or methyl,

D represents an oxygen atom or a radical of the formula -CO-, $-(CO)_a-NR^7$, $-(CH_2)_bS-$, $-(CH_2)_c-NR^8$ or -CH=CH-,
in which

a, b and c are identical or different and denote a number 0 or 1,

$R^7$ and $R^8$ are identical or different and denote hydrogen, methyl, ethyl, phenyl, acetyl or benzyl, the ring systems optionally being substituted up to 2 times identically or differently by nitro, fluorine, chlorine, bromine, trifluoromethyl or by straight-chain or branched alkyl or alkoxy each having up to 3 carbon atoms,

E and L are identical or different and represent hydrogen, fluorine, chlorine or bromine,

$R^1$ represents cyclopropyl, cyclobutyl, cyclopentyl or cyclohexyl or straight-chain or branched alkyl having up to 8 carbon atoms,

$R^2$ represents hydrogen or straight-chain or branched alkyl having up to 3 carbon atoms,

$R^3$ represents a radical of the formula,

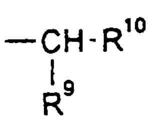

in which

$R^9$ denotes hydrogen or a radical of the formula $CH_2$-OH,

$R^{10}$ denotes phenyl which is optionally substituted up to 2 times identically or differently by hydroxyl, fluorine, chlorine, methyl or ethyl,

and their salts.

**4.** Hetero-linked phenylglycinolamides according to Claims 1 to 3 as medicaments.

**5.** Process for the preparation of hetero-linked phenylglycinolamides according to Claims 1 to 3, characterized in that carboxylic acids of the general formula (II)

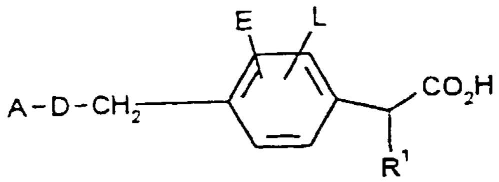

(II)

in which

A, D, E, L and $R^1$ have the meaning given above, are reacted, optionally with prior activation of the carboxylic acid function, with phenylglycinols of the general formula (III)

$$HR^2\text{-}N\text{-}R^3 \quad \text{(III)}$$

in which
$R^2$ and $R^3$ have the meaning indicated above,

if appropriate under a protective gas atmosphere, if appropriate in inert solvents, in the presence of a base and/ or auxiliary.

**6.** Medicaments containing at least one hetero-linked phenylglycinolamide according to Claims 1 to 3 and a pharmacologically acceptable formulation auxiliary.

**7.** Medicaments according to Claim 6 for the treatment of atherosclerosis.

**8.** Use of hetero-linked phenylglycinolamides according to Claims 1 to 3 for the production of medicaments.

**9.** Use according to Claim 8 for the production of antiatherosclerotic medicaments.

**10.** Use of hetero- and phenylglycinolamides according to Claims 1 to 3 for the production of medicaments for the reduction or complete inhibition of the formation and/or the release of Apo B-100-associated lipoproteins.

**11.** Use according to Claim 8 for the production of medicaments for the prevention and treatment of adiposity.